# EUROPEAN PATENT APPLICATION

(11) **EP 4 640 240 A1**
(43) Date of publication of application: **29.10.2025**
(21) Application number: 24171634.9
(22) Date of filing: 22.04.2024
(51) Int. Cl.: A61K 51/08, A61K 103/00

(54) **PEPTIDE-BASED RADIOPHARMACEUTICALS FOR NON-INVASIVE IMAGING OF THE FUNCTIONAL LIVER RESERVE**

(71) Applicant: Medizinische Universität Innsbruck, 6020 Innsbruck (AT)
(72) Inventor: HAUBNER, Ronald, 6020 Innsbruck (AT); ZIERKE, Maximilian Alexander, 6020 Innsbruck (AT); RANGGER, Christine, 6020 Innsbruck (AT)
(74) Representative: Grünecker Patent- und Rechtsanwälte PartG mbB

(57) **Abstract**

The present invention relates to peptide-based radiopharmaceuticals of formula (I) 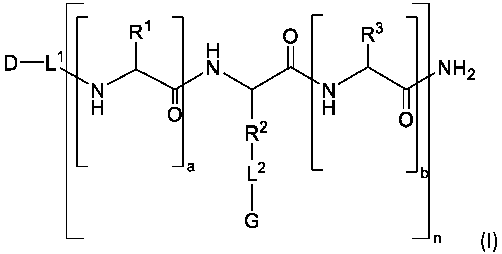 for non-invasive imaging of the functional liver reserve.

## Description

### Field of the Invention

The present invention relates to new and inventive compounds, especially compounds useful as SPECT (single-photon emission computed tomography) tracer or PET (positron emission tomography) tracer. The present invention further relates to a pharmaceutical or diagnostic composition comprising such a compound. The present invention further relates to a method of synthesizing the compound. The present invention further relates to a method of imaging hepatocytes, comprising a) contacting the hepatocyte with such a compound and b) visualizing the compound that is in contact with the hepatocyte. Also, the present invention relates to a method of determining functional hepatic reserve, comprising a) contacting the liver with such a compound, and b) visualizing the compound that is in contact with the liver. The present invention further relates to the use of the compound for imaging hepatocytes as well as to the use for determining functional hepatic reserve.

### Background of the Invention

The asialoglycoprotein receptor (ASGR) is a c-type lectin mainly expressed on the basolateral side of hepatocytes, where up to 500.000 receptors per cell can be found (D'Souza AA et al. J Control Release 2015;203:126-39) . In contrast, in the remainder of the body expression is low, making it a promising target for e.g. drug delivery into hepatocytes. The main physiological function of the ASGR is to maintain serum glycoprotein homeostasis by clearing of desialylated glycoproteins bearing D-galactose (Gal) or N-acetylgalactosamine (GaINAc) as terminal carbohydrates. Therefore, these glycoproteins are internalized via receptor-mediated endocytosis. This is initiated by binding of the glycoprotein to the ASGR, followed by the migration of the receptor-ligand complex to active internalization sites (clathrin coated pits) where corresponding vesicles are formed leading to the internalization of the complex. Subsequently, in the cell, the glycoprotein is degraded in the lysosome and the ASGR is recycled (Stockert RJ. et al. Physiol Rev 1995;75:591-609; de Graaf W et al. J Nucl Med 2010;51:742-52). The ASGR consists of two homologous subunits H1 and H2, which are responsible for Gal/GalNAc recognition (Lee YC et al. J Biol Chem 1983;258:199-202). Carbohydrate binding was elucidated by X-ray structure analysis of the carbohydrate recognition domain (CRD) of the H1 subunit (Meier M et al. J Mol Biol 2000;300:857-65).

Numerous monovalent and multivalent ligands for hepatocytes targeting via the ASGR have been developed. Multivalent ligands based on different scaffolds have been shown to achieve high binding affinity to the ASGR if tri- or tetraantennary ligands are used (Huang X et al. Bioconjugate Chem 2017;28:283-295; Zacco E et al. ACS Chem Biol 2015;10:2065-2072). In contrast, the preferred compounds of the present invention have more than three sugar residues.

Non-invasive methods allowing quantitative determination of the functional liver mass are of great interest for patient management in a diversity of clinical settings comprising liver surgery and liver transplantation (de Graaf Wet al. J Nucl Med 2010;51:742-52; Hoekstra LT et al. Ann Surg 2013;257:27-36; Kaibori M et al. Ann Nucl Med 2011;25:593-602) as well as diagnosis (Virgolini I et al.. Br J Cancer 1990;61:937-41; Kurtaran A et al. J Nucl Med 1995;36:1875-81) and treatment monitoring (Virgolini I et al. Br J Cancer 1993;68:549-54) of cancer. Additionally, it has been revealed that the evaluation of remnant liver function can help to discriminate different stages of alcoholic liver cirrhosis (Virgolini I et al. Nucl Med Commun 1991;12:507-17) and could be used to differentiate areas of steatosis, fibrosis and cholestasis (Bennink RJ et al. Semin Nucl Med 2012;42:124-37). Moreover, control of liver status before and during peptide receptor radionuclide therapy (PRRT) (Mansi L et al. Eur J Nucl Med Mol Imaging 2011;38:605-12) could lead to an optimized patient management. Further, patients who are potentially suitable for selective internal radiation therapy (SIRT) (D'Arienzo M et al. Nucl Med Commun 2012;33:633-40) may also benefit from such a diagnostic method, allowing stratifying patients according to their peri-interventional risk.

Due to the restricted expression, the ASGR is an optimal target structure for non-invasive monitoring of the liver function. [^{99m}Tc]Tc-diethylenetriamine-pentaacetic acid galactosyl human serum albumin (^{99m}Tc-GSA) (Kokudo N et al. Nucl Med Biol 2003;30:845-9) has been developed to image ASGR expression using single photon emission tomography (SPECT). It has been shown that ^{99m}Tc-GSA and dynamic SPECT allows estimation of regional hepatic function based on the determination of the ASGR density (Kudo M et al. Hepatology 1993;17:814-9; Vera DR et al. J Nucl Med 1996;37:160-4). To combine the superior performance of positron emission tomography (PET) compared with SPECT concerning imaging resolution and quantifying properties with the excellent properties of GSA in ASGR targeting, we developed a [⁶⁸Ga]Ga-labelled analogue (Haubner R et al. Eur J Nucl Med Mol Imaging 2016;43:2005-13). [⁶⁸Ga]Ga-DTPA-GSA showed comparable targeting properties as found for ^{99m}Tc-GSA, but lacks high metabolic stability. By replacing DTPA with 2-S-(4-isothiocyanatobenzyl)-1,4,7-triazacyclononane-1,4,7-triacetic acid (p-NCS-Bn-NOTA) [⁶⁸Ga]Ga-NOTA-GSA was developed, which demonstrated the desired high metabolic stability, which was even higher than that of the original ^{99m}Tc-labelled DTPA-GSA, allowing the use of pharmacokinetic modeling approaches for more detailed quantification (Haubner R et al. Mol Imaging Biol 2017;19:723-30). Moreover, it confirms the superiority of PET, which includes the possibility of time-resolved 3D volume determination, allowing accurate quantification of the tracer dynamics throughout the entire liver and enabling the reader to delineate exactly the volumes of interest and move from a rough estimation of liver function to a spatially resolved quantification. Despite the good imaging performance of human GSA-based radiotracers the translation of this class of compounds into clinical practice is limited due to regulations of biological products isolated from human material. The requirements in the production of a GMP-compliant labelling precursor, which are essential for use in humans, increased during the last years. Thus, despite the good imaging performance, the translation of this human serum albumin-based labelling precursor into clinical practice was not possible, indicating the need of radiolabelled low molecular weight, synthetic derivatives as galactose carrier. Therefore, particular research interest has arisen in the development of galactose-based synthetic small molecule radiotracers.

### Summary of the Invention

It is the objective of the present invention to provide peptide-based low molecular weight radiopharmaceuticals for the non-invasive determination of the asialoglycoprotein receptor expression, and in particular for determining functional hepatic reserve.

This problem is solved according to the present invention by the following embodiments and aspects of the invention.

In a first aspect, the present invention relates to a compound according to formula (I) 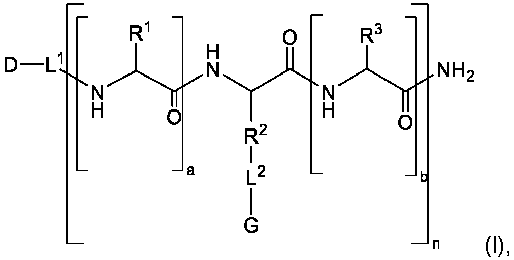 wherein R¹-R³, D, G, L¹, L², a, b and n are as defined below.

In a further aspect, the present invention relates to a pharmaceutical composition comprising the compound according to formula (I) and a pharmaceutically acceptable excipient.

In a further aspect, the present invention relates to a method of producing the compound according to formula (I), the method comprising synthesizing the compound.

In a further aspect, the present invention relates to a pharmaceutical or diagnostic composition comprising the compound according to formula (I) and at least one additive.

In a further aspect, the present invention relates to a method of imaging hepatocytes, comprising a) contacting the hepatocyte with a compound according to formula (I), and b) visualizing the compound that is in contact with the hepatocyte.

In a further aspect, the present invention relates to a method of determining functional hepatic reserve, comprising a) contacting the liver with the compound according to formula (I), and b) visualizing the compound that is in contact with the liver.

In a further aspect, the present invention relates to the use of the compound according to formula (I) for imaging hepatocytes.

In a further aspect, the present invention relates to the use of the compound according to formula (I) for determining functional hepatic reserve.

### Brief Description of the Drawings

The invention will be described in more detail in the following section and illustrated in accompanying figures.
**Figure 1** shows NODAGA-NonaLysan, the compound of Example 3.
**Figure 2** shows NOTA-NonaLysan, the compound of Example 4.
**Figure 3** shows NODAGA-GalNAc-NonaLysan, the compound of Example 5.
**Figure 4** shows SiFA-NonaLysan.
**Figure 5** shows the reaction scheme for preparing the compounds of Examples 1, 2, 3 and 5.
**Figure 6** shows a radio-HPLC of ⁶⁸Ga-labelled NODAGA-TriLysan, column: Dr. Maisch ReproSil Pur C18 AQ 150 x 4.6 mm, 5 µm, 120 Å; gradient: 5-25 % B in 15 min; solvent A: water + 0.1 % TFA (v/v); solvent B: MeCN + 0.1 TFA (v/v); flow: 1 mL/min.
**Figure 7** shows a radio-HPLC of ⁶⁸Ga-labelled NODAGA-HexaLysan, column: Dr. Maisch ReproSil Pur C18 AQ 150 x 4.6 mm, 5 µm, 120 Å; gradient: 5-25 % B in 15 min; solvent A: water + 0.1 % TFA (v/v); solvent B: MeCN + 0.1 TFA (v/v) flow: 1 mL/min.
**Figure 8** shows a radio-HPLC of ⁶⁸Ga-labelled NODAGA-NonaLysan, column: Dr. Maisch ReproSil Pur C18 AQ 150 x 4.6 mm, 5 µm, 120 Å; gradient: 5-25 % B in 15 min; solvent A: water + 0.1 % TFA (v/v); solvent B: MeCN + 0.1 TFA (v/v) flow: 1 mL/min.
**Figure 9** shows a radio-HPLC of ⁶⁸Ga-labelled NODAGA-GaINAc-NonaLysan, column: Dr. Maisch ReproSil Pur C18 AQ 150 × 4.6 mm, 5 µm, 120 Å; gradient: 5-25 % B in 15 min; solvent A: water + 0.1 % TFA (v/v); solvent B: MeCN + 0.1 TFA (v/v) flow: 1 mL/min.
**Figure 10a** shows biodistribution data (% ID/g) of [⁶⁸Ga]Ga-NODAGA-TriLysan (n = 3), [⁶⁸Ga]Ga-NODAGA-HexaLysan (n = 6), [⁶⁸Ga]Ga-NODAGA-NonaLysan (n = 9) and [⁶⁸Ga]Ga-NODAGA-GaINAc-NonaLysan (n = 9) comparative compound GSA in healthy BALB/c mice at 10, 30 and 60 min p.i.
**Figure 10b** shows biodistribution data (% ID/g) of [⁶⁸Ga]Ga-NODAGA-NonaLysan (n = 9) and [⁶⁸Ga]Ga-NODAGA-GaINAc-NonaLysan (n = 9) in healthy BALB/c mice and when blocked with 27.7 µmol *N*-acetylgalactose (both 30 min p.i.)
**Figure 11** shows PET/MR images of [⁶⁸Ga]Ga-NODAGA-NonaLysan. Image **a** shows a T2w-MRI signal, image **b** shows a representative frame of dynamic 60 min PET imaging, and image **c** is an overlay of images a and b.
**Figure 12** shows the concentration of [⁶⁸Ga]Ga-NODAGA-NonaLysan within a region of interest in liver and heart (reflects activity concentration in blood) measured over time from a dynamic scan (time-activity curve).

### Detailed Description of the Invention

All publications, including but not limited to patents, patent applications and scientific publications, cited in this description are herein incorporated by reference for all purposes as if each individual publication were specifically and individually indicated to be incorporated by reference.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various aspects and embodiments described therein, and it is to be understood that aspects and embodiments (and features therein) described under one subheading may be freely combined with aspects and embodiments (and features therein) described under another subheading. Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

The skilled person understands that the present invention is not limited to the embodiments explicitly mentioned above and below, but also includes combinations of aspects, embodiments or features that are not explicitly discussed herein, but can be implicitly derived.

The present invention relates to a compound of formula (I), or a pharmaceutically acceptable salt thereof, 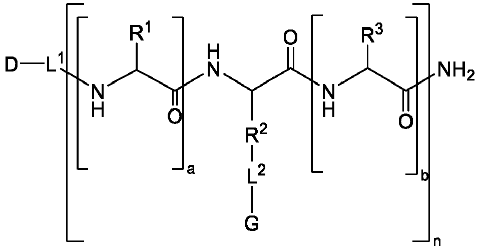 wherein
D is a chelating group or a prosthetic group or a [¹⁸F]F accepting group;
each instance of R¹ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or R¹ forms a 5-membered heterocycle with the alpha nitrogen, the heterocycle containing up to one additional heteroatom selected from O, N and S;
each instance of R² is independently selected from the group consisting of ^{#}-NH-(C(R⁴)₂)_{d}-, and ^{#}-(C=O)-(C(R⁴)₂)_{d}-,
   wherein ^{#} designates the attachment point to L²;
each instance of R³ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or R³ forms a 5-membered heterocycle with the alpha-nitrogen, the heterocycle containing up to one additional heteroatom selected from O, N and S;
each instance of R⁴ is independently selected from the group consisting of H, C₁₋₄-alkyl, and OH;
each instance of R⁵ is independently selected from the group consisting of 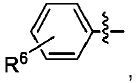 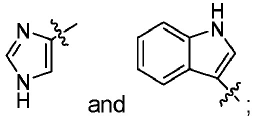
R⁶ is selected from the group consisting of H, Hal and OH;
L¹ is a linker selected from the group consisting of *-N(H)-(CH₂)ₓ-C(O)-, aspartic acid, glutamic acid, lysine, *-N(H)-(CH₂-CH₂-O)_{y}-(CH₂)_{z}-C(O)-, 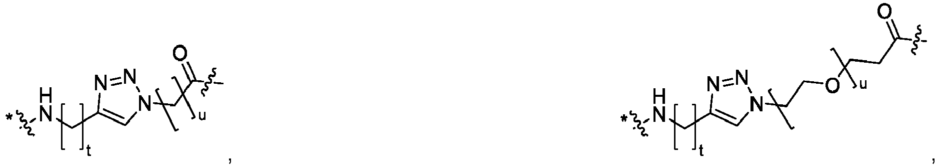 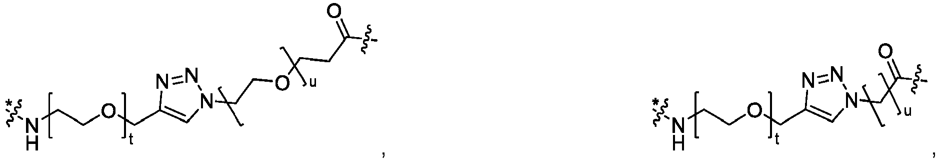 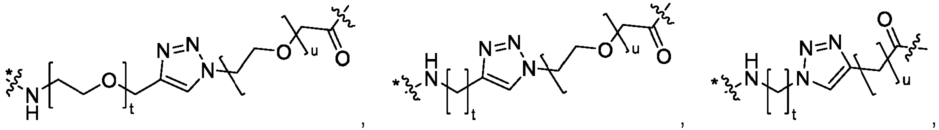 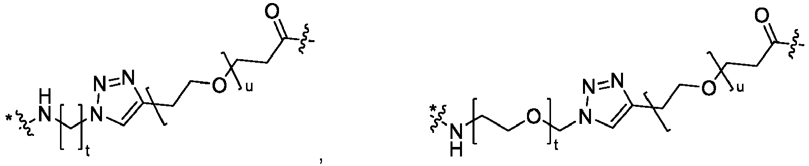 and 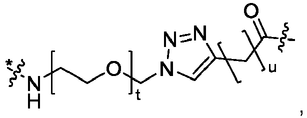 wherein * designates the attachment point to D;
L² is a linker selected from the group consisting of 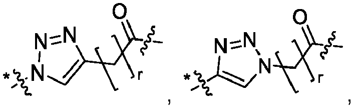 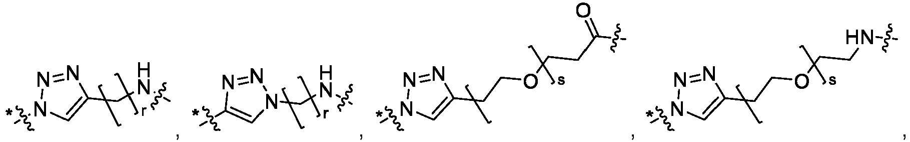 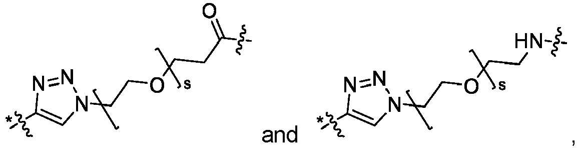 wherein * designates the attachment point to G;
G is Gal, GaINAc, or Lac;
a is 0-3;
b is 1-6; with the proviso that a + b ≤ 6;
d is independently selected from 0-6;
n is 3-15;
r is independently selected from 1-6;
s is 1-6;
u is independently selected from 1-6;
t is independently selected from 3-6;
x is 1-6;
y is 1-6; and
z is 1-2.

In some embodiments, D is a chelating group selected from the group consisting of NODAGA and derivatives thereof such as NODAGA, NOTA, DOTAGA, DO3A, HBED, DTPA and derivatives thereof such as NCS-DTPA, DFO and derivatives thereof such as p-NCS-Bz-DFO, FusC, DafC, CNAAZTA, NOPO, TRAP, DOTPI, DOTAZA, sacrophagine-based chelators, TE2A derivatives such as CB-TE2A, EDDA/HYNIC, pyrazole derivatives such as PZ1, N₃S-ligands such as MAG₃, MAS₃ or H-Asp-Lys-Cys, tetradentate nitrogen (N4) ligands such as 6-carboxy-1,4,8,11-tetraazaundecane, isonitril conjugates with an NS₃-COOH coligand (as found e.g. in ^{99m}Tc-N₃SCOOH-L2-RGD), and Tc-nitrido cores using Cys and phosphine coligands (e.g. PNP) for complexation.

In some embodiments, D is a chelating group selected from the group consisting of NODAGA and derivatives thereof such as NODAGA, NOTA, DOTAGA, DO3A, HBED, DTPA and derivatives thereof such as NCS-DTPA, DFO and derivatives thereof such as p-NCS-Bz-DFO, FusC, DafC, CNAAZTA, NOPO, TRAP, DOTPI, DOTAZA, sacrophagine-based chelators, and TE2A derivatives such as CB-TE2A.

In some embodiments, D is a chelating group selected from the group consisting of 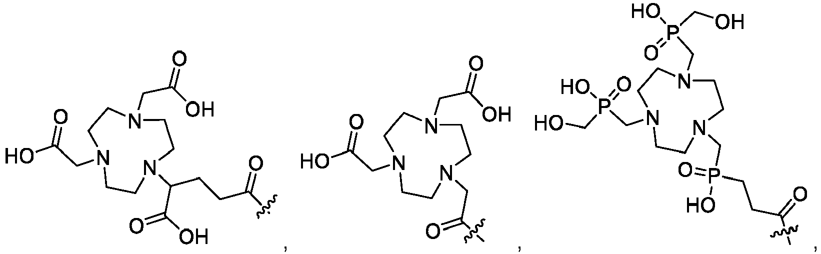 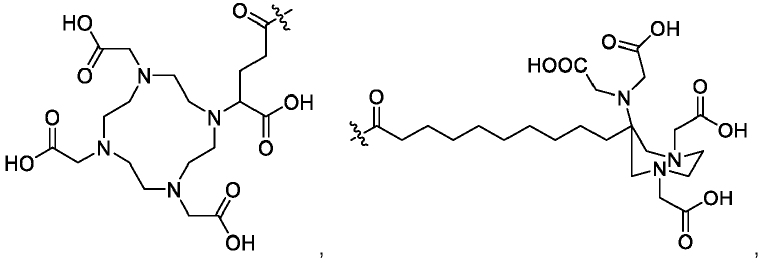 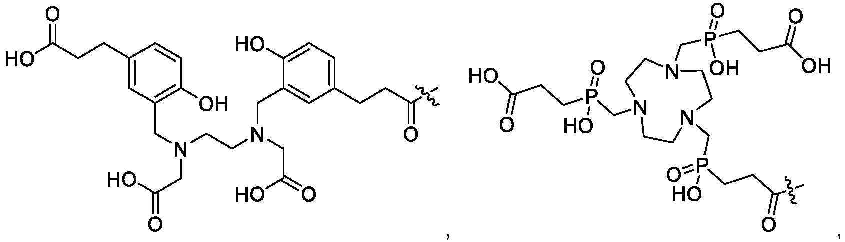 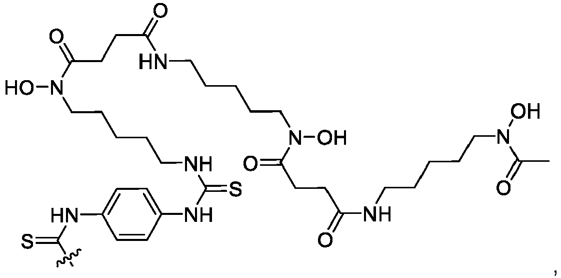 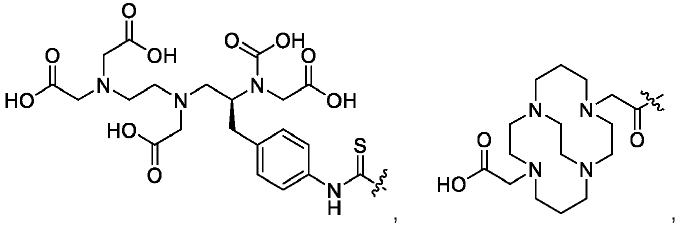 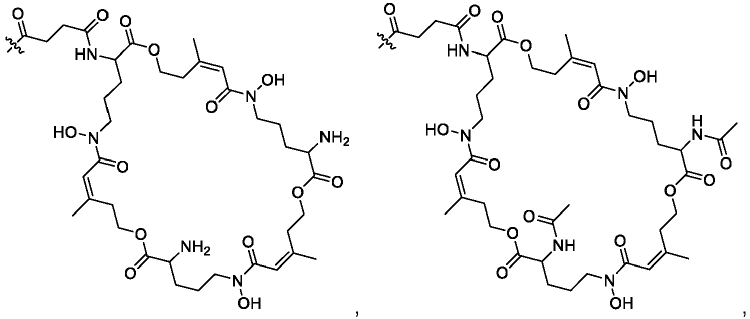 and

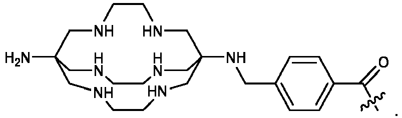

In a preferred embodiment, D is a chelating group selected from

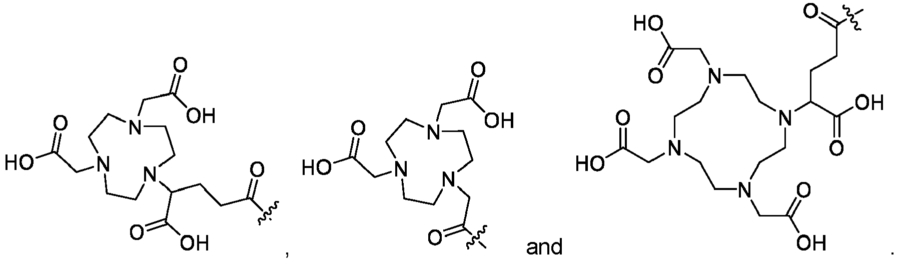

Most preferably, the chelator D is

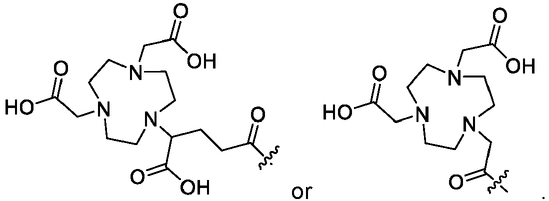

In some embodiments, D is a chelating group selected from the group consisting of EDDA/HYNIC, pyrazole derivatives such as PZ1 with CO as coligand(s), N₃S-ligands such as MAG₃, MAS₃ or H-Asp-Lys-Cys, tetradentate nitrogen (N4) ligands such as 6-carboxy-1,4,5,11-tetraazaundecane, isonitril conjugates with an NS₃-COOH coligand (as found e.g. in ^{99m}Tc-N₃SCOOH-L2-RGD), and Tc-nitrido cores using Cys and phosphine coligands (e.g. PNP) for complexation .

In some embodiments, D is a chelating group selected from the group consisting of

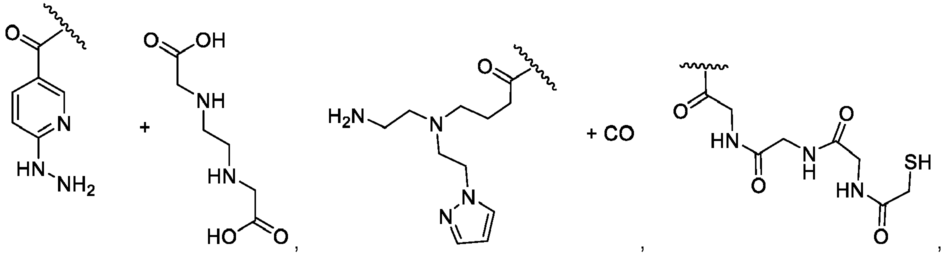

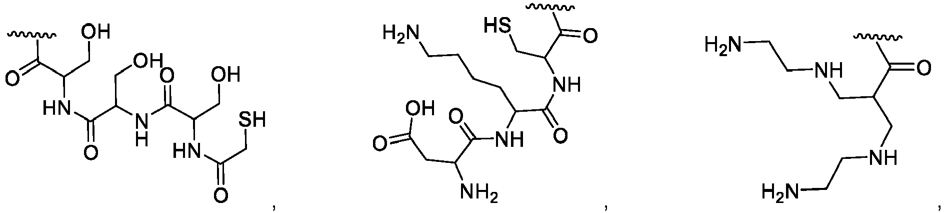

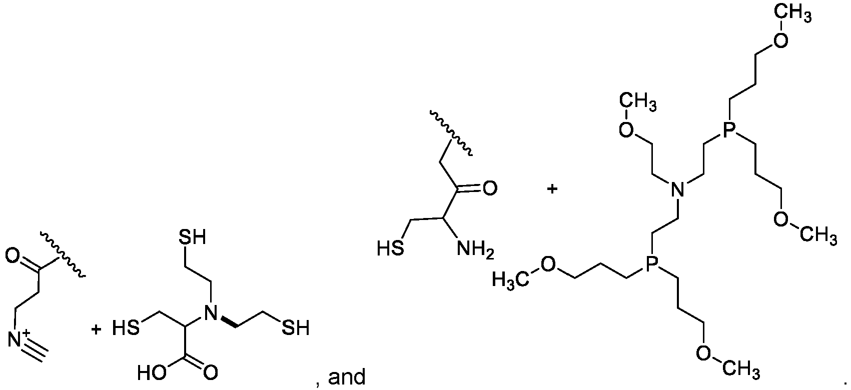

In a preferred embodiment, D is a chelating group selected from the group consisting of 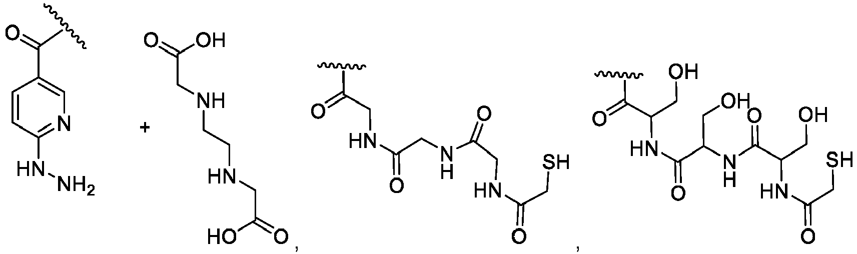 and

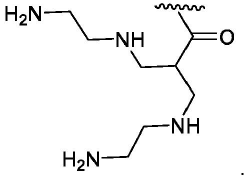

In a more preferred embodiment, D is

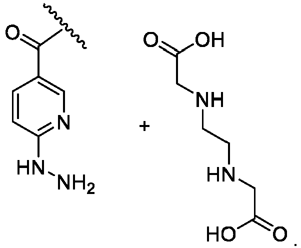

In some embodiments, R¹ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or R¹ forms a 5-membered heterocycle with the alpha nitrogen, the heterocycle containing up to one additional heteroatom selected from O, N and S. Preferably, the heterocycle contains only the alpha-nitrogen as a heteroatom.

In the context of the present invention, said alpha-nitrogen designates the nitrogen atom adjacent to the carbon atom to which the substituent R¹ is attached.

In some embodiments, R¹ is an amino acid side-chain in the L- or D-configuration. More preferably, R¹ is a proteinogenic amino acid side-chain in the L-configuration. In the context of the present invention, proteinogenic amino acids include glycine (Gly), alanine (Ala), valine (Val), leucine (Leu), isoleucine (Ile), methionine (Met), phenylalanine (Phe), tyrosine (Tyr), tryptophan (Trp), serine (Ser), Threonine (Thr), asparagine (Asn), glutamine (Gln), cysteine (Cys), selenocysteine (Sec), proline (Pro), arginine (Arg), histidine (His), lysine (Lys), aspartic acid (Asp) and glutamic acid (Glu), preferably in the L-configuration. In some embodiments, the proteinogenic amino acid is selected from the group consisting of glycine, alanine, valine, leucine, serine, methionine, cysteine and lysine, preferably in the L-configuration. In a preferred embodiment, the proteinogenic amino acid is selected from the group consisting of glycine, alanine, valine and leucine, preferably in the L-configuration. Most preferably, the proteinogenic amino acid is glycine.

In a preferred embodiment, R¹ is independently H, CH₃-(C(R⁴)₂)_{d}-, HS-(C(R⁴)₂)_{d}-, CH₃-S-(C(R⁴)₂)_{d}-, HO-(C(R⁴)₂)_{d}-, or NH₂-(C(R⁴)₂)_{d}-, wherein R⁴ is independently selected from H, CH₃ and OH, and d is independently selected from 0, 1 or 2. In a more preferred embodiment, R¹ is independently H or CH₃-(C(R⁴)₂)_{d}-, wherein R⁴ is independently selected from H, C₁₋₂-alkyl and OH. Most preferably, R¹ is H.

In some embodiments, R² is independently selected from the group consisting of ^{#}-NH-(C(R⁴)₂)_{d}-, and ^{#}-(C=O)-(C(R⁴)₂)_{d}-,wherein ^{#} designates the attachment point to L².

In a preferred embodiment, R² is ^{#}-NH-(C(R⁴)₂)_{d}- and L² is 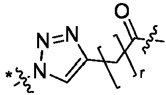 or 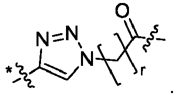 In a more preferred embodiment, R² is ^{#}-NH-(CH₂)₄- and L² is

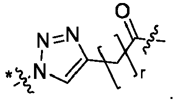

In a further equally preferred embodiment, R² is ^{#}-(C=O)-(C(R⁴)₂)_{d}- and L² is 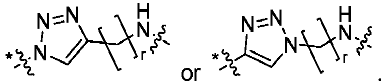 In a more preferred embodiment, R² is *-(C=O)-(CH₂)- or ^{#}-(C=O)-(CH₂)₂- and L² is

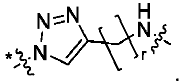

In some embodiments, R³ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or R³ forms a 5-membered heterocycle with the alpha nitrogen, the heterocycle containing up to one additional heteroatom selected from O, N and S. Preferably, the heterocycle contains only the alpha-nitrogen as a heteroatom.

In the context of the present invention, said alpha-nitrogen designates the nitrogen atom adjacent to the carbon atom to which the substituent R³ is attached.

In some embodiments, R³ is an amino acid residue in the L- or D-configuration. More preferably, R³ is a proteinogenic amino acid side-chain in the L-configuration. In the context of the present invention, proteinogenic amino acids include glycine (Gly), alanine (Ala), valine (Val), leucine (Leu), isoleucine (Ile), methionine (Met), phenylalanine (Phe), tyrosine (Tyr), tryptophan (Trp), serine (Ser), Threonine (Thr), asparagine (Asn), glutamine (Gln), cysteine (Cys), selenocysteine (Sec), proline (Pro), arginine (Arg), histidine (His), lysine (Lys), aspartic acid (Asp) and glutamic acid (Glu), preferably in the L-configuration. In some embodiments, the proteinogenic amino acid is selected from the group consisting of glycine, alanine, valine, leucine, serine, methionine, cysteine and lysine, preferably in the L-configuration. In a preferred embodiment, the proteinogenic amino acid is selected from the group consisting of glycine, alanine, valine and leucine, preferably in the L-configuration. Most preferably, the proteinogenic amino acid is glycine.

In a preferred embodiment, R³ is independently H, CH₃-(C(R⁴)₂)_{d}-, HS-(C(R⁴)₂)_{d}-, CH₃-S-(C(R⁴)₂)_{d}-, HO-(C(R⁴)₂)_{d}-, or NH₂-(C(R⁴)₂)_{d}-, wherein R⁴ is independently selected from H, CH₃ and OH, and d is independently selected from 0, 1 or 2. In a more preferred embodiment, R³ is independently H or CH₃-(C(R⁴)₂)_{d}-, wherein R⁴ is independently selected from H, C₁₋₂-alkyl and OH. Most preferably, R³ is H.

In some embodiments, L¹ is a linker selected from the group consisting of *-N(H)-(CH₂)ₓ-C(O)-, aspartic acid, glutamic acid, lysine, *-N(H)-(CH₂-CH₂-O)_{y}-(CH₂)_{z}-C(O)-, 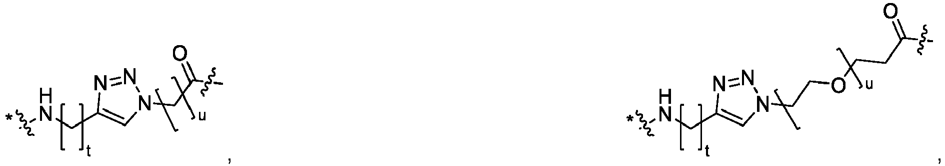 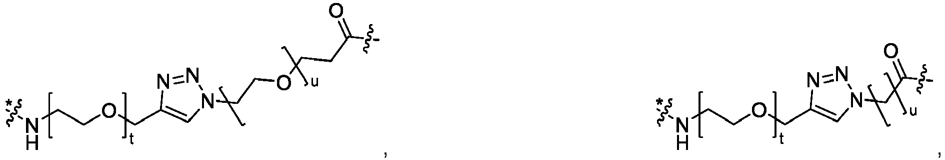 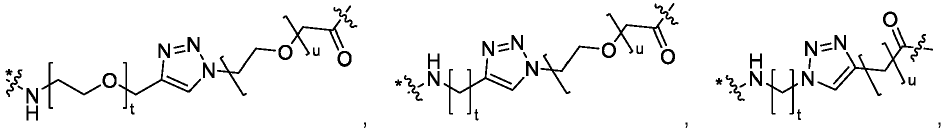 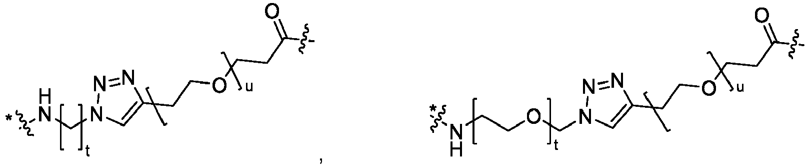 and 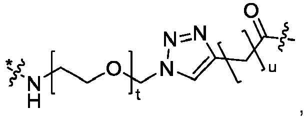 wherein* designates the attachment point to D.

In a preferred embodiment, L¹ is selected from the group consisting of *-N(H)-(CH₂)ₓ-C(O)-, *-N(H)-(CH₂-CH₂-O)_{y}-(CH₂)_{z}-C(O)-, 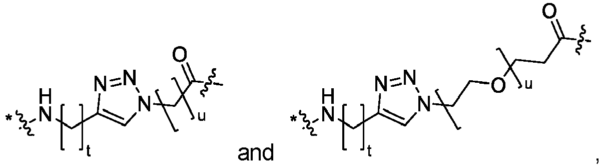 wherein each of x, y, z, and u is selected from 1, 2 or 3 and t is selected from 3, 4 or 5. In a more preferred embodiment, L¹ is *-N(H)-(CH₂)ₓ-C(O)-, wherein x is 2 or 3, more preferably, x is 3.

In some embodiments, L² is a linker selected from the group consisting of 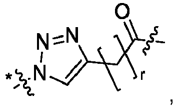 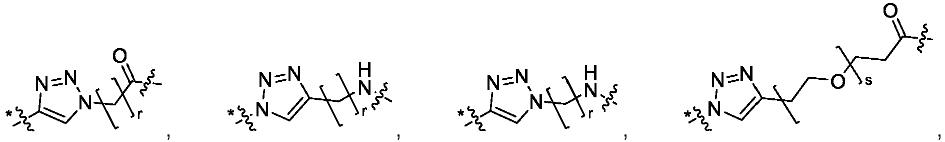 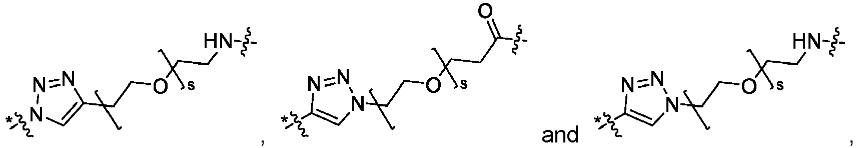 wherein * designates the attachment point to G.

In a preferred embodiment, L² is selected from the group consisting of

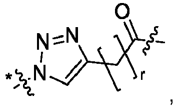

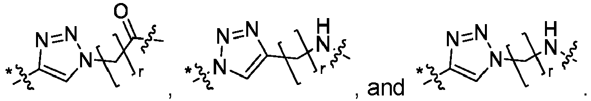

In a preferred embodiment, L² is selected from the group consisting of 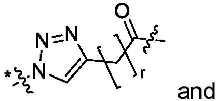 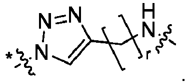 More preferably, L² is

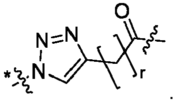

In preferred embodiments, n is 3-15, 4-15, 5-15 or 6-15. In a more preferred embodiment, n is 3, 6, 9, 12 or 15. In a further more preferred embodiment, n is 3, 6 or 9. Most preferably, n is 6 or 9.

In some embodiments, G is Gal, GaINAc, or Lac. In a preferred embodiment, G is Gal or GaINAc.

In some embodiments, a is 0-3. In a preferred embodiment, a is 0 or 1. More preferably, a is 0.

In some embodiments, b is 0-6. In a preferred embodiment, b is 1-4. More preferably, b is 2.

In some embodiments, d is independently selected from 0-6. In a preferred embodiment, d is independently selected from 1-4. More preferably, d is independently selected from 1-3.

In some embodiments, r is independently selected from 1-6. In a preferred embodiment, r is independently selected from 2-5. More preferably, r is independently selected from 3-5. Most preferred, r is 4.

In some embodiments, s is 1-6. In a preferred embodiment, s is 2-5.

In some embodiments, u is independently selected from 1-6. In a preferred embodiment, u is independently selected from 2-5.

In some embodiments, t is independently selected from 3-6. In a preferred embodiment, t is independently 3 or 4.

In some embodiments, x is 1-6. In a preferred embodiment, x is 2 or 3. More preferably, x is 3.

In some embodiments, y is 1-6. In a preferred embodiment, y is 1-4. More preferably, y is 1-3.

In some embodiments, z is 1-2.

In a preferred embodiment, D is a chelating group or a prosthetic group or a [¹⁸F]F accepting group; R¹ is independently from each other selected from the group consisting of H and CH₃-; R² is independently from each other selected from the group consisting of ^{#}-NH-(C(R⁴)₂)_{d}-, wherein R⁴ is H, d is 3-4 and ^{#} designates the attachment point to L²; R³ is independently from each other selected from the group consisting of H and CH₃-; L¹ is *-N(H)-(CH₂)ₓ-C(O)- or *-N(H)-(CH₂-CH₂-O)_{y}-(CH₂)_{z}-C(O)-, wherein x is 2-4, y is 1-4 and z is 1-2 and * designates the attachment point to D; 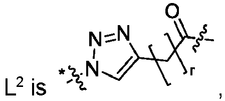
wherein r is 3-5 and * designates the attachment point to G;
G is Gal, GaINAc, or Lac; and
n is 3, 6, or 9.
In a preferred embodiment, the compound of formula (I) is

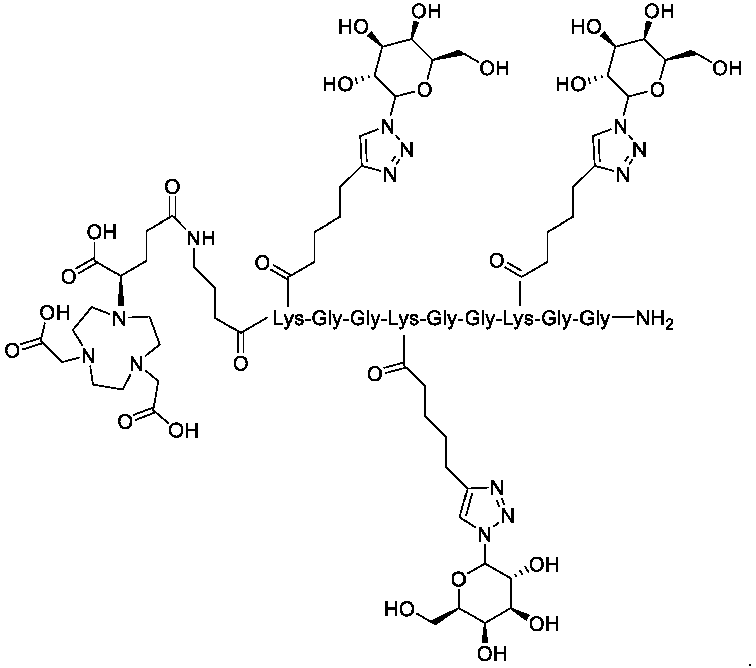

Preferably, the amino acids of the peptide backbone in the above structural formula are in the L-configuration.

In a preferred embodiment, the compound of formula (I) is

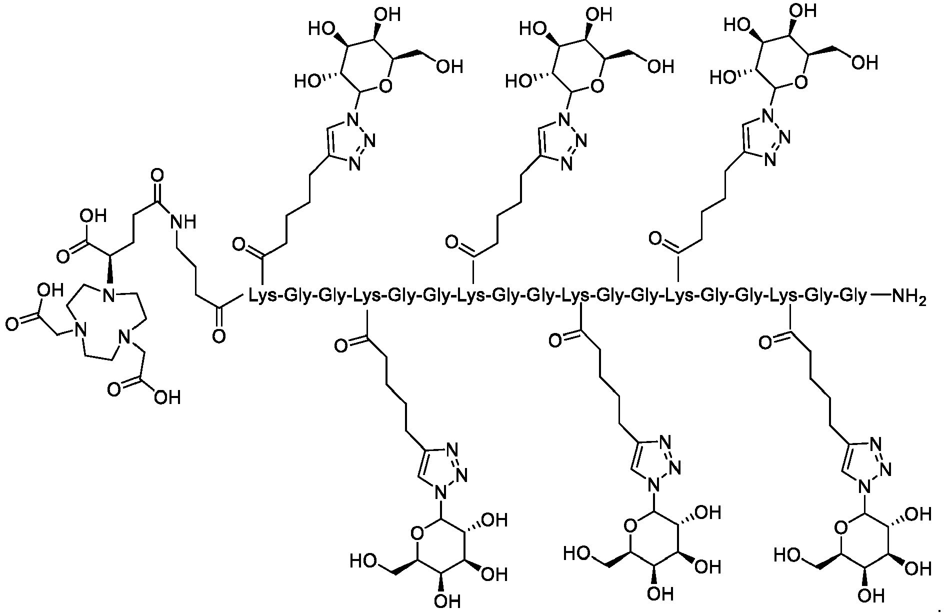

Preferably, the amino acids of the peptide backbone in the above structural formula are in the L-configuration.

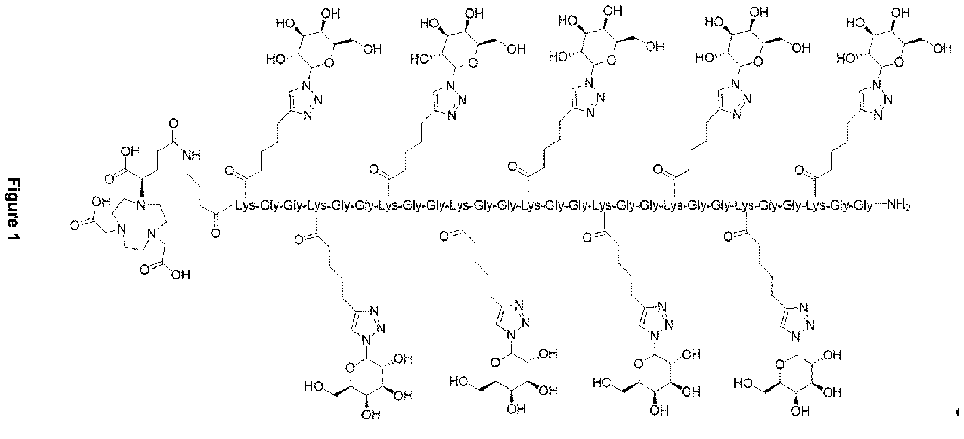

In a preferred embodiment, the compound of formula (I) is the compound shown in Fig. 1. Preferably, the amino acids of the peptide backbone in the structural formula of Fig. 1 are in the L-configuration.

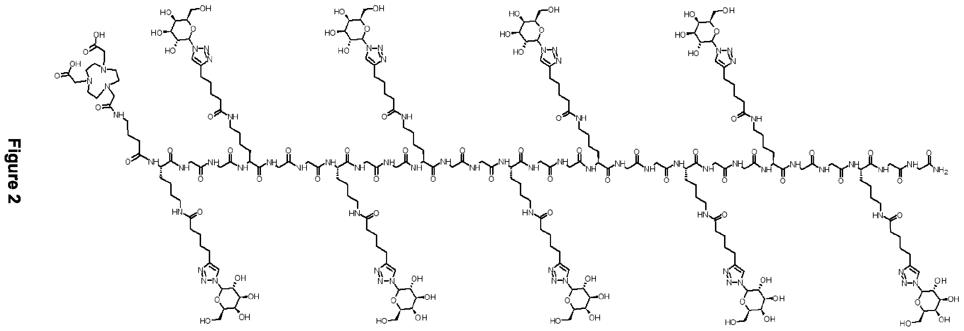

In a preferred embodiment, the compound of formula (I) is the compound shown in Fig. 2. Preferably, the amino acids of the peptide backbone in the structural formula of Fig. 2 are in the L-configuration.

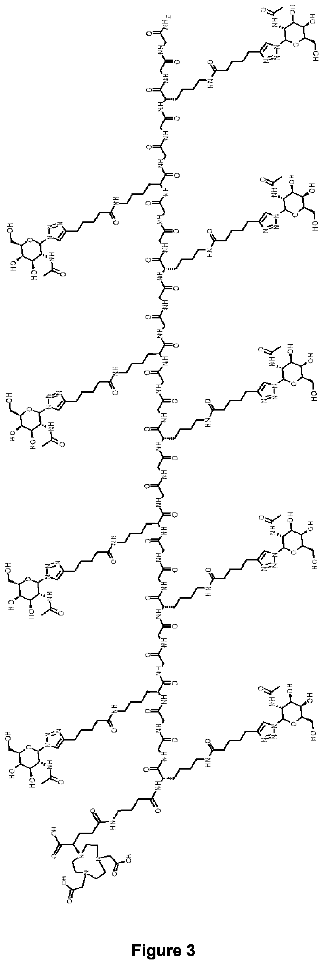

In a preferred embodiment, the compound of formula (I) is the compound shown in Fig. 3. Preferably, the amino acids of the peptide backbone in the structural formula of Fig. 3 are in the L-configuration.

D is a chelating group (chelator), a prosthetic group or a [¹⁸F]F accepting group.

The term "chelator" or "chelating group" is known in the art and refers to organic compounds that are polydentate ligands that form two or more coordinate bonds with a radionuclide. The chelator may contain different donor groups for metal complexation such as oxygen, nitrogen, sulphur, (carboxyl, phosphonate, hydroxamate, amine, thiol, thiocarboxylate or derivatives thereof) and comprises acyclic and macrocyclic chelators such as polyaminopolycarboxylic ligands. The introduction of the radionuclide into the chelator is typically performed after conjugation of the chelator to the remainder of the molecule.

The term "prosthetic group" is known in the art and refers to a bifunctional labelling agent that is a small organic molecule that can be easily radiolabelled, for example with a radionuclide, and conjugated to a second molecule, e.g. a biomolecule such as a sugar containing molecule. Generally, prosthetic groups are conjugated to amines, thiols or carboxylic acid functions present in the second molecule. Also conjugation via click chemistry is possible.

The term "[¹⁸F]F accepting group" refers to a group that can be selectively covalently modified with [¹⁸F]F.

In some embodiments, D is
a) a chelating group which chelates [⁶⁸Ga]Ga, [¹⁸F]AlF, [⁸⁹Zr]Zr, [⁶⁴Cu]Cu, [⁸⁶Y]Y, [^{99m}Tc]Tc or [¹¹¹In]In; or
b) a prosthetic group which is labelled with radionuclides of halogens selected from [¹²⁴I]I, [¹²³I]I, [⁷⁵Br]Br, [⁷⁶Br]Br or [¹⁸F]F; or
c) a [¹⁸F]F accepting group which is modified with [¹⁸F]F.

In some embodiments, D is
a) a chelating group which chelates [⁶⁸Ga]Ga, [¹⁸F]AlF, [⁸⁹Zr]Zr, [⁶⁴Cu]Cu, or [⁸⁶Y]Y; or
b) a prosthetic group which is labelled with radionuclides of halogens selected from [¹²⁴I]I, [¹²³I]I, [⁷⁵Br]Br, [⁷⁶Br]Br or [¹⁸F]F; or
c) a [¹⁸F]F accepting group which is modified with [¹⁸F]F.

In some embodiments, D is a chelating group and coordinates to (chelates) a radionuclide.

The radionuclide is preferably selected from [⁶⁸Ga]Ga, [¹⁸F]AlF, [⁸⁹Zr]Zr, [⁶⁴Cu]Cu, [^{99m}Tc]Tc, [¹¹¹In]In and [⁸⁶Y]Y, more preferably [⁶⁸Ga]Ga or [¹⁸F]AlF, most preferably [⁶⁸Ga]Ga.

In some embodiments, the chelator is selected from the group consisting of NODAGA (1,4,7-triazacyclononane-1-glutaric acid-4,7-diacetic acid), NOTA (1,4,7-triazacyclononane-1,4,7-triacetic acid), DOTAGA (1,4,7,10-Tetraazacyclododecane-1-glutaric acid-4,7,10-triacetic acid), DO3A (1,4,7,10-tetraazacyclododecane-1,4,7-triacetic acid), HBED (N,N'-di(2-hydroxybenzyl)-ethylenediamine-N,N'-diacetic acid), NCS-DTPA (S-2-(4-isothiocyanatobenzyl)-diethylene-triamine pentaacetic acid), p-NCS-Bz-DFO (N1-hydroxy-N1-(5-(4-(hydroxy(5-(3-(4-isothiocyanatophenyl)thioureido)pentyl)amino)-4-oxobutanamido)pentyl)-N4-(5-(N-hydroxyacetamido)pentyl)succinamide), FusC (Fusarinine C), DafC (Diacetyl-Fusarinine C), CNAAZTA (1,4-bis(carboxymethyl)-6-[bis(carboxymethyl)]amino-6-(9-carboxynonyl)-perhydro-1,4-diazepine), NOPO (3-(((4,7-bis((hydroxy(hydroxymethyl)-phosphoryl)methyl)-1,4,7-triazonan-1-yl)methyl)(hydroxy)-phosphoryl)propanoic acid), TRAP (3,3',3"-(((1,4,7-triazonane-1,4,7-triyl)tris(methylene))tris(hydroxyphosphoryl))tripropanoic acid, DOTPI (1,4,7,10-tetraazacyclododecane-1,4,7, 10-tetrakis[methylene(2-carboxyethylphosphinic acid)), DOTAZA (1,4,7,10-Tetra-azacyclododecan-1,4,7,10-tetra-azidoethylacetic acid), sacrophagine-based chelators, TE2A derivatives such as CB-TE2A (2,2'-(1,4,8,11-tetraazabicyclo[6.6.2]hexadecane-4,11-diyl)diacetic acid), HYNIC (hydrazinonicotinic acid) with EDDA (ethylenediaminediacetic acid) as coligand, PZ1 (3,5-(CH₃)₂-pyrazole(CH₂)₂N((CH₂)₃COOH)(CH₂)₂NH₂), N₃S systems as MAG₃ (mercaptoacetyl-Gly₃), MAS₃ (mercaptoacetyl-Ser₃), N4-ligands as 6-carboxy-1,4,5,11-tetraazaundecane, isonitril conjugates (introduced e.g. via 4-isocyano-butanoic acid) with an NS₃-COOH coligand (as found e.g. in ^{99m}Tc-N₃SCOOH-L2-RGD), and Tc-nitrido cores using Cys and phosphine coligands (e.g. PNP (N(CH₂CH₂OCH₃)(CH₂CH₂P(CH₂CH₂CH₂OCH₃)₂)₂)) for complexation.

The radionuclide is preferably a positron-emitting isotope of a metal or halogen suitable for PET imaging. Suitable radionuclides to be coordinated to the chelating group include but are not limited to [⁸⁹Zr]Zr, [⁴⁴Sc]Sc, [⁴⁵Ti]Ti, [⁵¹Mn]Mn, [⁶⁴Cu]Cu, [⁶¹Cu]Cu, [⁶³Zn]Zn, [⁶⁸Ga]Ga, [¹¹C]C [¹²⁴I]I, [⁸⁶Y]Y, and [¹⁸F]AIF. In some embodiments, the radionuclide is a metal ion such as [⁶⁸Ga]Ga, [⁸⁹Zr]Zr, [⁴⁴Sc]Sc, [⁶⁴Cu]Cu, or [⁸⁶Y]Y, more preferably [⁶⁸Ga]Ga, or [⁶⁴Cu]Cu. Most preferably, the radionuclide is [⁶⁸Ga]Ga. In some other embodiments, the radionuclide is a halogen such as [¹²⁴I]I or a halide such as [¹⁸F]AlF, most preferably [¹⁸F]AIF. In some embodiments, the radionuclide is selected from [⁶⁸Ga]Ga and [¹⁸F]AIF.

In an equally preferred embodiment, the radionuclid is a gamma ray-emitting isotope of a metal or halogen suitable for SPECT imaging. Suitable radionuclides to be coordinated to the chelating group include but are not limited to [^{99m}Tc]Tc and [¹¹¹In]In, more preferably [^{99m}Tc]Tc. A suitable halogen is [¹²³I]I.

It is to be understood that various factors may be included in the selection of a suitable imaging isotope. Such factors include a sufficient half-life of the isotope to allow preparation of a diagnostic composition prior to administration to the patient, and a sufficient remaining half-life to yield sufficient activity to allow non-invasive measurement by PET or SPECT. The isotope should have a sufficiently short half-life to limit patient exposure to unnecessary radiation. Particularly suitable isotopes to be coordinated by the chelating groups of the present invention are [⁶⁸Ga]Ga and [¹⁸F]AIF for PET and [^{99m}Tc]Tc and [¹¹¹In]In for SPECT.

Preferred prosthetic groups of the present invention are labelled with radionuclides of halogens such as [¹²⁴I]I, [¹²³I]I, [⁷⁵Br]Br, [⁷⁶Br]Br or [¹⁸F]F, preferably [¹⁸F]F. The introduction of the radionuclide into the prosthetic group is typically performed before conjugation of the prosthetic group to the remainder of the molecule.

In some embodiments the radiolabelled prosthetic group is selected from a group comprising but not limited to 4-[¹⁸F]fluorophenacyl bromide, N-succinimidyl-4-[¹⁸F]fluorobenzoate ([¹⁸F]SFB), N-succinimidyl-4-([¹⁸F]fluoromethyl)benzoate, 4-[¹⁸F]fluorobenzaldehyde, ¹⁸F-6-fluoropyridine-3-carboxylic acid 2,3,5,6-tetrafluorophenyl ester ([¹⁸F]F-Py-TFP), silicon-containing building blocks such as N-succinimidyl 3-(di-tert-butyl[¹⁸F]fluorosilyl)benzoate ([¹⁸F]SiFB), carbohydrate-based prosthetic groups, such as [¹⁸F]fluoro-deoxyglucose, preferably 2-[¹⁸F]fluoro-2-deoxyglucose ([¹⁸F]FDG), and [¹⁸F]fluoro-deoxymannose, preferably [¹⁸F]2-fluoro-2-deoxymannose, or derivatives thereof, maleimide-based and heterocyclic methylsulfone-based ¹⁸F-synthons, ¹⁸F-labelled prosthetic groups such as ¹⁸F-azides or ¹⁸F-alkynes permitting labelling via click chemistry, ¹⁸F-labelled organo trifluoroborates and [¹⁸F]fluoropyridines. The preferred prosthetic group is ¹⁸F-6-fluoropyridine-3-carboxylic acid 2,3,5,6-tetrafluorophenyl ester (F-Py-TFP). The linkers are designed to have a N-terminal amino function allowing labelling via any prosthetic group, which reacts with an amine.

In some embodiments a chelator-based labelling approach using aluminium-fluoride ([¹⁸F]AIF) is applied for radiofluorination.

In some embodiments, D is a [¹⁸F]F accepting group. In some embodiments, direct labelling of the compound of the present invention is achieved by attaching a [¹⁸F]F accepting group to the remainder of the molecule. For instance, N,N,N-trimethyl-5-[(2,3,5,6-tetrafluorophenoxy)carbonyl]pyridin-2-aminium trifluoromethanesulfonate can be conjugated to the amino function of R group under release of tetrafluorophenol. The trimethylammonium group acts as a leaving group and can be replaced by, e.g. [¹⁸F]F.

Also contemplated is the use of para-substituted di-tert-butylfluorosilylbenzene structural motif, which is known as the silicon-fluoride acceptor (SiFA), for incorporating radioactive [¹⁸F]F into the compound of the present invention. Labelling with [¹⁸F]F is performed by isotopic exchange of [¹⁹F]F from SiFA with [¹⁸F]F.

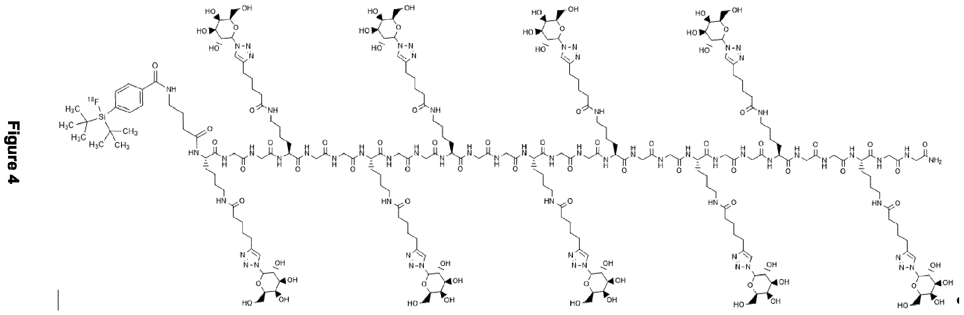

In some embodiments, the compound has the structure as shown in Fig. 4. Preferably, the amino acids of the peptide backbone in the structural formula of Fig. 4 are in the L-configuration. In some embodiments, the fluorine of the tert-butylfluorosilylbenzene moiety is [¹⁸F]F.

The sugar moiety G is galactose (Gal, D-galacto-hexopyranose), galactose with an acetylated amino residue at position 2 (GaINAc, 2-(acetylamino)-2-deoxy-D-galactose), or lactose (Lac). More preferably, G is Gal or GaINAc. The sugar moiety may be connected to the linker L² via an O-glycosidic linkage, an N-glycosidic linkage, an S-glycosidic linkage or a C-glycosidic linkage. In a preferred embodiment, the linkage is O-glycosidic.

The present invention further relates to pharmaceutical compositions, preferably for diagnostic use, comprising the compound described herein and at least one pharmaceutically acceptable additive.

The present invention further provides a method for synthesizing the compound described herein.

The method comprises the steps of
(1) peptide-coupling of amino acid residues;
(2) conjugating L² to R²;
(3) activating L² to allow coupling with a modified sugar residue;
(4) conjugating L¹-D to the remainder of the molecule.

The present invention also relates to a method of imaging cells as described herein. The method of imaging cells described herein makes use of the compound described herein.

The present method of imaging cells makes us of established methods of imaging. Preferably, positron emission tomography (PET) is used for imaging the cells. PET is a nuclear imaging methology that detects pairs of gamma rays emitted indirectly by a positron-producing radionuclide. Compared to other radioimaging methologies such as Single Photon Emission Computed Tomography (SPECT), PET is known to show higher sensitivity, better spatial resolution, greater signal to noise ratio, and superior tracer quantification in both preclinical and clinical applications.

The present invention also includes strategies to produce radiopharmaceuticals which allow the use of SPECT. Despite some limitations in physical imaging properties this imaging technique is the most widely spread technique and thus, allows the introduction also where no PET imaging facility is available.

Combining the functional imaging obtained by PET or SPECT, which depicts the spatial distribution of metabolic or biochemical activity in the body, with computer tomography (CT) allows to more precisely align or correlate the results with the anatomy of the body. Combination of PET with magnetic resonance imaging (MRI) combines the exquisite structural and functional characterization of tissue provided by MRI with the extreme sensitivity of PET imaging of metabolism and tracking of uniquely labeled cell types or cell receptors.

Radiopharmaceuticals such as the compounds of the present invention are of great interest for patient management in a diversity of clinical settings comprising liver surgery and liver transplantation, diagnosis and treatment monitoring of cancer, as well as other liver diseases like alcoholic liver cirrhosis and liver fibrosis. The optimal liver function assessment will allow a precise evaluation of the different liver segments aimed at better predicting liver failure following major liver surgery.

The compounds of the present invention overcome the drawbacks of the human GSA-based radiotracers, e.g. ^{99m}Tc-GSA, in the art because they can be easily prepared and put to practice in clinical routine. Surprisingly, the present inventors have found that the compounds of the present invention show higher affinity for the target receptors compared to the GSA-based radiotracer. Without being bound by theory, it is thought that due to the lower molecular mass, elimination from the body is faster, resulting, at least for later time points, in improved liver to organ ratios compared with GSA-based radiotracer such as ^{99m}Tc-GSA.

In one aspect, the present invention provides a method of imaging hepatocytes, comprising a) contacting the hepatocyte with a compound of the present invention and b) visualizing the compound that is in contact with the hepatocyte.

In a further aspect, the present invention provides a method of determining functional hepatic reserve, comprising a) contacting the liver with the compound of the present invention, and b) visualizing the compound that is in contact with the liver.

In some embodiments, the visualizing is performed via positron emitting computer tomography (PET).

In some embodiments, the visualizing is performed via single-photon emission computed tomography (SPECT).

The methods of the invention may be performed *in vivo* or *in vitro.*

The contacting of the hepatocyte or the contacting of the liver with the compound can be achieved, for example, by administering the compound to a patient. Administration to the patient can be by injection, in particular by intravenous injection.

For administration to a patient, the compound is formulated in a suitable dosage form. One suitable dosage form is a pharmaceutical or diagnostic composition, comprising at least one additive, such as saline.

In some embodiments, the molar activity of the radiolabelled compound ranges from about 50 MBq/nmol up to about 33 GBq/nmol.

In some embodiments, if D is a chelator group and the radionuclide is [⁶⁸Ga]Ga, the molar activity of the radiolabelled compound is about 50-500 MBq/nmol. In preferred embodiments, the molar activity of the radiolabelled compound is about 50-450 MBq/nmol, about 50-400 MBq/nmol, about 100-500 MBq/nmol, about 100-450 MBq/nmol, about 100-400 MBq/nmol, or about 120-400 MBq/nmol. In a more preferred embodiment, the molar activity of the radiolabelled compound is about 100-400 MBq/nmol.

In some embodiments, if D is a chelator group and the radionuclide is [^{99m}Tc]Tc, the molar activity of the radiolabelled compound is about 5-50 GBq/µmol. In preferred embodiments, the molar activity of the radiolabelled compound is about 5-45 GBq/µmol, about 5-40 GBq/µmol, about 10-50 GBq/µmol, about 10-45 GBq/µmol, about 10-40 GBq/µmol, about 12-40 GBq/µmol or about 15-40 GB1/µmol. In a more preferred embodiment, the molar activity of the radiolabelled compound is about 10-40 GBq/µmol.

In some embodiments, if D is a prosthetic group and the radionuclide is [¹⁸F]F or if D is an [¹⁸F]F acceptor group, the molar activity of the radiolabelled compound ranges from 1,000 MBq/nmol up to 33 GBq/nmol. In preferred embodiments, the molar activity of the radiolabelled compound is about 3-20 GBq/nmol, about 3.5-10 GBq/nmol, about 3.5-5 GBq/nmol, or about 3.5-4.5 GBq/nmol. In a more preferred embodiment, the molar activity of the radiolabelled compound is about 3.5-5 GBq/nmol.

In some embodiments, if D is a chelator group and the radionuclide is [¹⁸F]AlF, the molar activity of the radiolabelled compound ranges from 10 MBq/nmol up to 2 GBq/nmol.

In some embodiments, if the radiolabelled compound is produced via isotopic exchange reaction, the molar activity is about 50-500 MBq/nmol, more preferably about 50-450 MBq/nmol.

### Examples

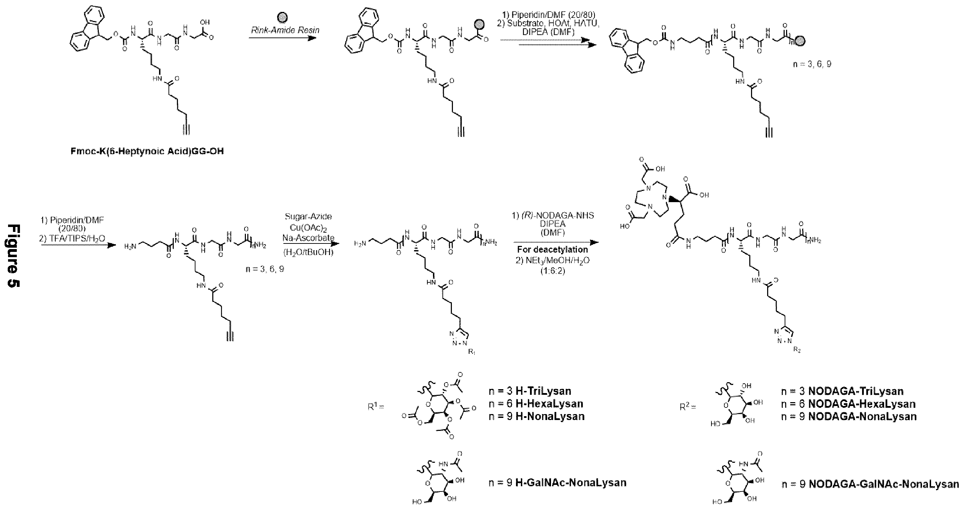

The compounds of the present invention can be prepared by a combination of methods known in the art including the procedures described in the reaction scheme 1 shown in Fig. 5. The reaction scheme is meant only to exemplify and does not limit the invention.

In a first step, the tripeptide of an amino acid sequence Fmoc-Lys(Dde)-Gly-Gly is generated on the chloro-trityl chloride resin occupied by glycine. The protective group Dde on the lysine is then removed. The 6-heptinic acid is then conjugated to the free amino function of the lysine. After cleavage of the trityl chloride resin, the tripeptide is coupled to a rink-amide resin (necessary to generate an amide C-terminal at the end). Then the N-terminal Fmoc protecting group is removed and, depending on n, (n-1) further tripeptide building blocks are conjugated. Finally, Fmoc-gamma-aminobutyric acid N-terminal is coupled to the peptide. After cleavage of the Fmoc protecting group and cleavage from the resin, the sugar-azide building block is conjugated via a "click" reaction. In the final step, the chelator is linked to the gamma-aminobutyric acid via the amino function.

### General procedures for solid phase peptide chemistry

### Loading of fragments onto a rink amide resin (GP1)

Rink amide resin (70 - 100 mg; δ = 0.65 mmol/g) was weighed into a 20 mL syringe equipped with a pp-frit inlet and was swollen in DMF for 1 hour. Removal of the first Fmoc-protection group was accomplished by incubating the resin for 1 hour with a mixture of 20 % Pip/DMF (vol/vol). The resin was washed with DMF (8 x 4 mL) and a solution of 0.70 eq Fmoc-K(6-heptynoic acid)GG-OH, 1.00 eq HOAt, 1.00 eq HATU and 4.50 eq DIPEA in DMF was added. After 24 hours of incubation time the resin was washed with DMF (3 × 4 mL) and all non-reacted active sites were capped by adding a mixture of acetic anhydride/pyridine (3:2) (vol/vol) for 30 min. The resin was washed once again with DMF (3 × 4 mL) and was ready to use for the next step.

### On-resin peptide bond formation for single amino acids (GP2a)

In a 5 mL glass vial HOAt (2.0 eq), HATU (2.0 eq) and the respective amino acid (2.0 eq) were dissolved in 3 mL of DMF with stirring. DIPEA (6.0 eq) was added and the solution was stirred for another 5 min at room temperature. The mixture was added to the resin and the reaction was allowed to continue for 2 hours.

### Fragment Coupling (GP2b)

Fmoc-K(6-heptynoic acid)GG-OH (0.8 eq) was weighed into 5 mL glass vial together with 1.0 eq HOAt and 1.0 eq HATU. DMF (2 mL) and DIPEA (4.50 eq) were added and the solution was stirred for 5 min at room temperature. This mixture was then added to the resin and the reaction was allowed to continue for 3 hours.

### Fmoc-Removal (GP3)

The resin was treated with a mixture of 20 % Pip/DMF (vol/vol) (1 × 5 min, 1 × 15 min) and was washed with DMF afterwards. (8 × 6 mL/g resin).

### On-resin Dde-deprotection (GP4)

For on resin Dde-deprotection in the presence of Fmoc-groups the resin was treated with a solution of Imidazole (0.92 g/g resin) and hydroxylamin hydrochloride (1.26 g/g resin) in 5 mL of NMP and 1 mL of DMF. The solution was added to the resin over a duration of 3 hours and the resin was washed with DMF (3 × 6 mUg resin) afterwards.

### Capping of unreacted amines (GP5)

The resin was treated for 30 min with 5 mL of a freshly prepared acetic anhydride/pyridine (3:2) solution. Afterwards the resin was washed 3 times with DMF.

### Cleavage of peptides from the resin (GP6)

The resin was washed thoroughly with DCM (3 × 4 mL) before 3 mL of a cleavage cocktail containing TFA/TIPS/H₂O (95/2.5/2.5) (vol/vol/vol) were added. After 45 min the solution containing the crude peptide was transferred into a 15 mL round bottom flask and 2 mL of new cleavage cocktail were added to the syringe. This step was repeated three times in total. The combined fractions were reduced under a stream or argon and either precipitated in ice-cold diethylether or directly lyophilized in a mixture of H₂O/*t*BuOH (1:1) (vol/vol).

### Preparation of intermediate Fmoc-K(6-Heptynoic Acid)-GG-OH

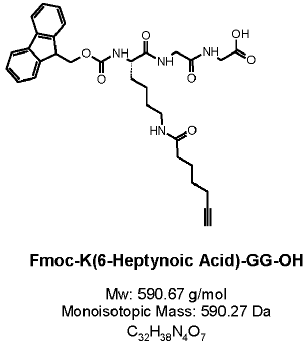

Assembly of the alkyne tagged GGK-motif, which serves as the general building block, was achieved via solid phase peptide synthesis starting with 500 mg (550 µmol) of glycin-loaded CTC-resin (δ = 1.10 mmol/g). Coupling of glycin and Dde-protected L-lysine was performed accordingly to a general procedure *(GP2a & GP3)*. On-resin Dde-removal was carried out using imidazol and hydroxylammoniumchloride *(GP4)*. Attachment of 6-heptynoic acid to the lysine sidechain was accomplished using HOAt (1.2 eq), HATU (1.2 eq) and DIPEA (3.0 eq) over the course of 3.5 hours. Cleavage of the motif from the resin was done according to *GP6.* Purification via semi-preparative HPLC (40-60 % MeCN/0.1%ACN in 25 min) and subsequent lyophilization yielded 316 mg (536 µmol, 87 %) of a colourless solid.

**RP-HPLC** (20-80 % MeCN/0.1%ACN in 15 min) t_{R} = 9.0 min (52 % MeCN/0.1%ACN).

MALDI-MS (m/z) = 591.5 [M+H]⁺, 613.5 [M+Na]⁺, 629.5 [M+K]⁺, 651.4 [M+Na+K]⁺.

### Example 1: Synthesis of NODAGA-TriLysan

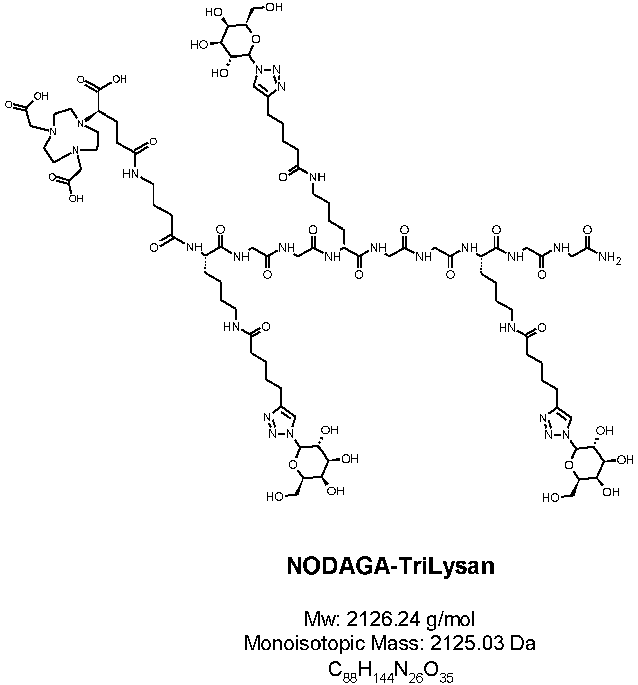

Fmoc-K(6-Heptynoic Acid)-GG-OH was loaded onto a rink amide resin (GP1), followed by a Fmoc-deprotection step (GP2). Assembly of all building blocks was performed accordingly to *GP2b* and GP3. For coupling of Fmoc-protected γ-amino butyric acid (Fmoc-GABA) only 1.0 eq of HOAt, HATU and 4.5 eq of DIPEA were used. Cleavage of the peptide from the resin followed GP6.

For galactosylation the crude peptide (45.7 mg, 33.5 µmol, 1.0 eq) was dissolved in 100 µL H₂O/*t*BuOH (1:1) (vol/vol) and mixed with 1,3,4,6-tetra-*O*-acetyl-2-azido-2-deoxy-D-galactopyranose (45 mg, 120 µmol, 3.6 eq) in 450 µL of hot H₂O/*t*BuOH/MeOH (1:1:1) (vol/vol/vol). Next, aqueous solutions of Cu(OAc)₂ (40.2 µmol, 1.2 eq) as well as sodium ascorbate (198 mg, 1 mmol, 30.0 eq) in a minimum amount of water were added. After 1 hour at 60 °C the galactosylated peptide was purified via semipreparative HPLC (32-46 % B in 25 min) and lyophilized subsequently. For attachment of the chelator, 5 mg (3.2 µmol, 1.0 eq) of this intermediate were reacted with NODAGA-NHS (9 mg, 12.8 µmol, 4.0 eq) in 450 µL dry DMSO. The pH was adjusted to 8-9with 22 µL (128 µmol, 40 eq) DIPEA. After2 hours at room temperature the reaction was stopped by addition of 1 mL of Millipore water followed by removal of all volatiles *in vacuo.* For deacetylation the crude residue was treated over night with 3 mL of NEt₃/MeOH/H₂O (1:6:2). Final purification via semipreparative HPLC (5-25 % MeCN/0.1%ACN in 25 min) followed by lyophilization yielded 2.2 mg (1 µmol, 3 %) of a colourless solid.

**Analytical HPLC** (ReproSil Pur, 5-25 % MeCN/0.1%ACN in 15 min, 1.0 mL/min) t_{R} = 11.0 min (18 % MeCN/0.1%ACN).

**MALDI-MS** (m/z) = 2147.2 [M+Na]⁺.

### Example 2: Synthesis of NODAGA-HexaLysan

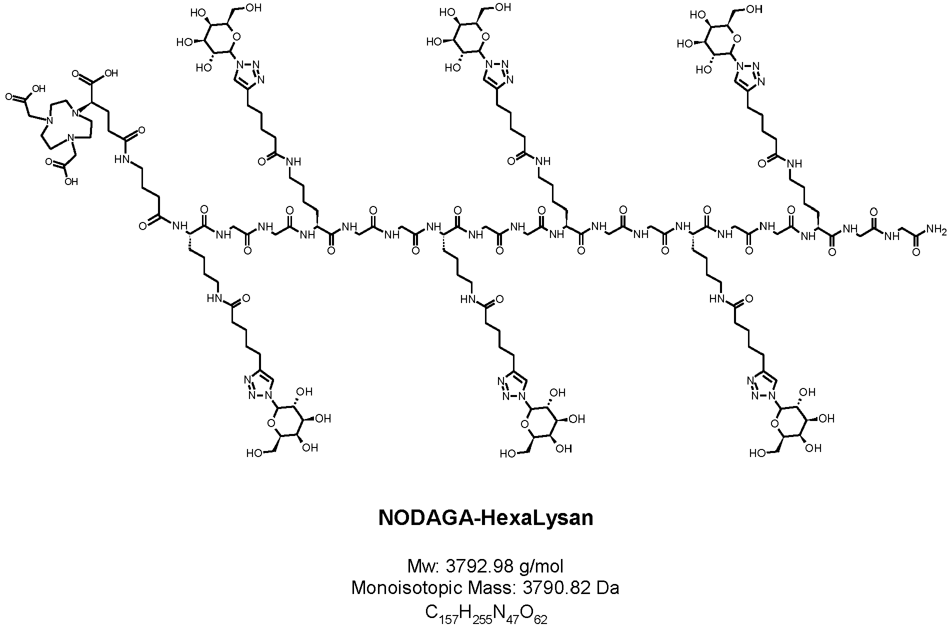

Synthesis of the peptidic backbone followed the same principle as outlined for NODAGA-TriLysan with some exceptions. After each coupling a capping step was performed *(GP5)* to avoid formation of truncated peptide species. Subsequent to coupling of Fmoc-GABA (GP2b & GP3), the deprotected peptide was cleaved from the resin and directly lyophylized (GP6).

For galactosylation the crude peptide (45 mg, 20 µmol, 1.0 eq) was dissolved in 500 µL H₂O/*t*BuOH (1:1) and a solution of 1,3,4,6-Tetra-*O*-acetyl-2-azido-2-deoxy-D-galactopyranose (53.7 mg, 144 µmol, 7.2 eq) in hot H₂O/*t*BuOH/MeOH (1:1:1) (vol/vol/vol) was added. Next, solutions of Cu(OAc)₂ (4.8 mg, 24 µmol, 1.2 eq) and sodium ascorbate (158 mg, 800 µmol, 40.0 eq) in a small amount of water were added and the resulting mixture was incubated for 1 h at 60 °C. After centrifugation (3000 rpm, 5 min) the supernatant was removed, syringe filtered and the fully galactosylated peptide was isolated via semipreparative HPLC (43-55 % MeCN/0.1%ACN in 25 min). For attachment of the chelator, 7.1 mg (1.5 µmol, 1.0 eq) of this intermediate were reacted with NODAGA-NHS (4.8 mg, 6.6 µmol, 4.3 eq) in 450 µL dry DMSO. The pH was adjusted to 8-9 with 10.5 µL (60 µmol, 40 eq) DIPEA. After 2 hours at room temperature the reaction was stopped by addition of 1 mL of Millipore water followed by removal of all volatiles *in vacuo.* For subsequent deacetylation the residue was treated over night with 3 mL of NEt₃/MeOH/H₂O (1:6:2) (vol/vol/vol). Removal of all volatiles and purification via semipreparative RP-HPLC (12-15 % MeCN/0.1%ACN) followed by lyophilization yielded 1.2 mg (316 nmol, 2 %) of a colourless solid.

**Analytical HPLC** (ReproSil Pur, 5-25 % MeCN/0.1%ACN in 15 min, 1.0 mL/min) t_{R} = 11.8 min (19 % MeCN/0.1%ACN).

**ESI-MS** (m/z) = 3793.82 [M+H]⁺.

### Example 3: Synthesis of NODAGA-NonaLysan (compound shown in Fig. 1)

Synthesis of the peptidic backbone followed the same principle as outlined for NODAGA-TriLysan with some exceptions. After each coupling a capping step was performed *(GP5)* to avoid formation of truncated peptide species. Subsequent to coupling of Fmoc-GABA (GP2b & GP3), the deprotected peptide was cleaved from the resin and directly lypohilized (GP6).

For galactosylation, the crude peptide (48.4 mg, 19.2 µmol, 1.0 eq) was dissolved in 500 µL H₂O/*t*BuOH (1:1) (vol/vol) and a solution of 1,3,4,6-Tetra-*O*-acetyl-2-azido-2-deoxy-D-galactopyranose (77.3 mg, 207 µmol, 10.8 eq) in hot H₂O/*t*BuOH/MeOH (1:1:1) (vol/vol/vol) was added. Next, solutions of Cu(OAc)₂ (4.6 mg, 23 µmol, 1.2 eq) and sodium ascorbate (153 mg, 768 µmol, 40.0 eq) in a small amount of water were added and the resulting mixture was incubated for 1 h at 60 °C. After centrifugation (3,000 rpm, 5 min) the supernatant was removed, syringe filtered and the fully galactosylated peptide was isolated via semipreparative HPLC (43-55 % MeCN/0.1%ACN in 25 min). For attachment of the chelator, 6.8 mg (1.0 µmol, 1.0 eq) of this intermediate were reacted with NODAGA-NHS (3 mg, 4.2 µmol, 4.2 eq) in 450 µL dry DMSO adjusted to pH 8-9 with 7 µL (40 µmol, 40 eq) DIPEA. After 2 hours at room temperature the reaction was stopped by addition of 300 µL of millipore water, followed by removal of all volatiles *in vacuo.* For subsequent deacetylation the residue was treated over night with 3 mL of NEt₃/MeOH/H₂O (1:6:2) (vol/vol/vol). All volatiles were removed *in vacuo* and the residue was purified via semipreparative HPLC (12-15 % MeCN/0.1%ACN). Subsequent lyophilization yielded 5 mg (916 nmol, 5 %) of a colourless solid.

**Analytical HPLC** (ReproSil Pur, 5-25 % MeCN/0.1%ACN in 15 min, 1.0 mL/min) t_{R} = 12.3 min (20 % MeCN/0.1%ACN).

**ESI-MS** (m/z) = 1820.88 [M+3H]³⁺, 1365.91 [M+4H]⁴⁺, 1092.93 [M+5H]⁵⁺.

### Example 4: Synthesis of NOTA-NonaLysan (compound shown in Fig. 2)

Synthesis of the peptidic backbone followed the same principles as for NODAGA-NonaLysan (Example 3). For attachment of the chelator the nonameric peptide (10 mg, 1.4 µmol, 1.0 eq) was reacted with NOTA-NHS (4.18 mg, 6.33 µmol, 4.4 eq) in 450 µL of dry DMSO adjusted to pH 8-9 with 11 µL (63.4 µmol, 44 eq) of DIPEA. After 2 hours at rt the reaction was stopped by addition of 600 µL of Millipore water followed by removal of all volatiles *in vacuo.* For subsequent deacetylation the residue was treated over night with 3 mL of (NEt₃/MeOH/H₂O) (1:6:2). Removal of all volatiles and purification via semipreparative RP-HPLC (12-15 % B) followed by lyophilization yielded 2.6 mg (463 nmol, 33 %) of a colourless solid.

**Analytical HPLC** (ReproSil Pur, 5-25 % MeCN/0.1%ACN in 15 min, 1.0 mL/min) t_{R} = 12.2 min (20 % MeCN/0.1%ACN).

**MALDI-MS** (m/z) = 5384.61 [M]⁻, 5446.73 [M+Na+K]⁻.

### Example 5: Synthesis of NODAGA-GaINAc-NonaLysan (compound shown in Fig. 3)

### Step 1: Synthesis of H-GaINAc-NonaLysan

Synthesis of the peptidic backbone followed the same principle as outlined for NODAGA-TriLysan with some exceptions. After coupling of Fmoc-GABA (GP2b & GP3) the deprotected peptide was cleaved from the resin (GP6). For N-acetylgalactosylation the crude peptide (38.2 mg, 11 µmol, 1.0 eq) was dissolved in 400 µL of *t*BuOH/H₂O and mixed with an aqueous solution of β-GalNAc-azide (25 mg, 101 µmol, 9.2 eq). Next, solutions of Cu(OAc)₂ (2.6 mg, 13.2 µmol, 1.2 eq) and Sodium Ascorbate (87 mg, 440 µmol, 40.0 eq) in a minimum amount of water were added. The mixture was incubated for1 hour at 60 °C directly injected into semipreparative HPLC (12-15 % B in 25 min). Lyophilization yielded 7.6 mg (1.4 µmol, 13 %) of a colourless solid.

**Analytical HPLC** (ReproSil Pur, 5-25 % MeCN/0.1%ACN in 15 min, 1.0 mL/min) t_{R} = 12.3 min (20 % MeCN/0.1%ACN).

**ESI-MS** (m/z) = 5472.72 [M+H]⁺.

### Step 2: Synthesis of NODAGA-GaINAc-Nonalysan

H-GaINAc-NonaLysan (750 µg, 134 nmol, 1.0 eq), prepared in step 1, was reacted with NODAGA-NHS (392 µg, 537 nmol, 4.0 eq) in 90 µL of dry DMSO adjusted to pH 8-9 with 0.5 µL (2.8 µmol, 20 eq) of DIPEA. After 2 hours at rt the reaction mixture was diluted ten-fold with Millipore water and directly subtracted to purification via analytical RP-HPLC (14 % B isocratic). Lyophilization of the product fraction yielded 250 µg (42 nmol, 31 %) of a colourless solid.

**Analytical HPLC** (ReproSil Pur, 5-25 % MeCN/0.1 % TFA in 15 min, 1.0 mL/min) t_{R} = 12.9 min (21 % MeCN/0.1 % TFA).

**ESI-MS** (m/z) = 5829.85 [M+H]⁺.

### Example 6: ⁶⁸Ga-labelling of NODAGA-conjugated peptides

A previously published procedure for labelling of NODAGA-conjugates with ⁶⁸Ga was applied with minor modifications from (Knetsch et al. Eur J Nucl Med Mol Imaging, 2011**.** Five µL (1mM, 5 nmol) of precursor were mixed with 100 µL of a 1 M Na-acetate buffer (pH = 5). Subsequently 550 µL of ⁶⁸Ga-eluate (80-100 MBq) were added and the solution was incubated for 15 min at 95 °C. The radiochemical purity was determined using radio-HPLC and radio-TLC.

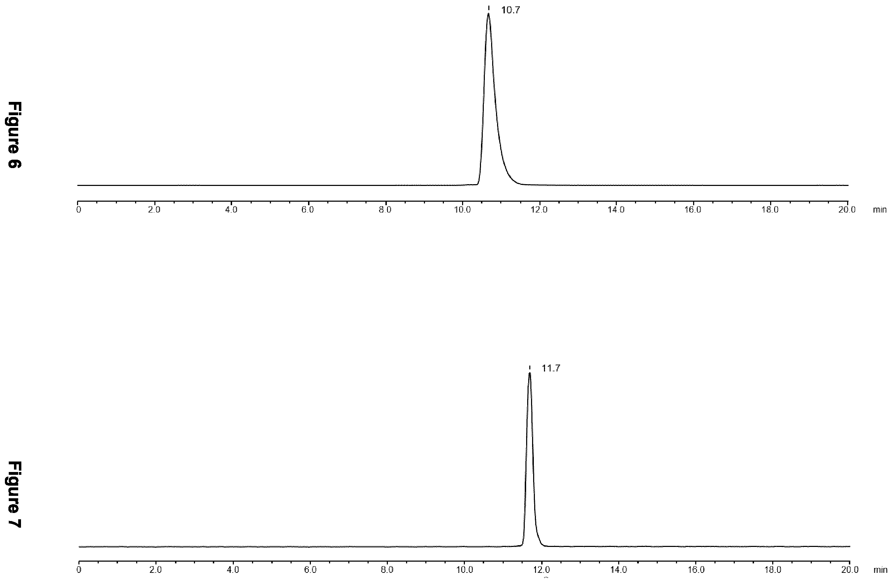

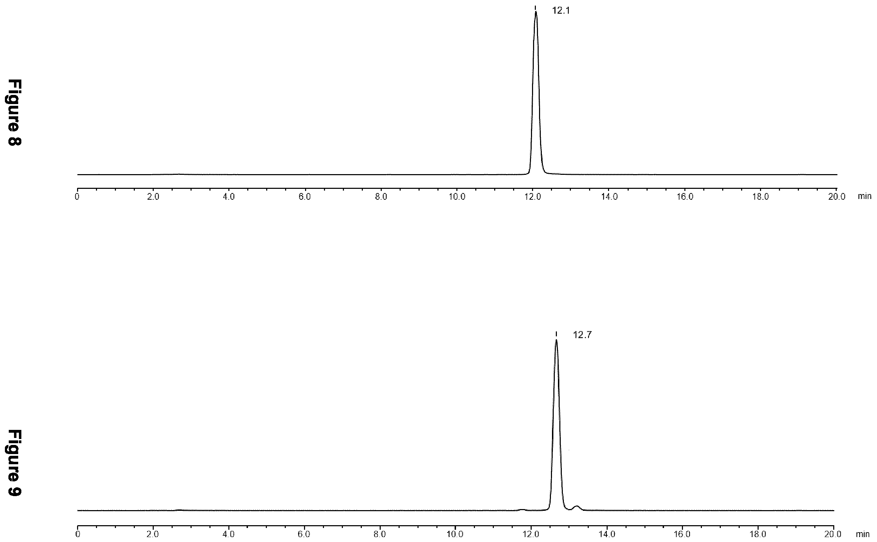

The radio-HPLC diagrams of NODAFA-conjugated peptides are shown in Fig. 6 (⁶⁸Ga-labelled NODAGA-TriLysan), Fig. 7 (⁶⁸Ga-labelled NODAGA-HexaLysan), Fig. 8 (⁶⁸Ga-labelled NODAGA-NonaLysan) and Fig. 9 (⁶⁸Ga-labelled NODAGA-GaINAc-NonaLysan).

### Example 7: In vitro evaluation

### Partition coefficient and protein binding

For determination of logarithmic partition coefficients (*logD*) *n*-Octanol (500 µL) and PBS (500 µL) were pipetted into eight 1.5 mL Protein Low-Bind Eppendorf tubes followed by addition of 300 kBq of labelled compound. The vials were vortexed for 5 min at 1,500 1/min and then centrifuged for 5 min at 20,000 rcf (Eppendorf Centrifuge 5424). Aliquots of 100 µL were taken out of each phase and the amount of activity was quantified in a γ-counter (Perkin Elmer 2480 Wizard2 3"; Waltham, Massachusetts, USA).

Protein binding was determined for different time points (2 min, 30 min, 60 min, 120 min) in triplicates using Sephadex MicroSpin G-50 columns (GE-Healthcare, Chicago, Illinois, USA). The columns were spun initially at 20,000 rcf for 1 min to remove the storage buffer. Then approx. 5 MBq of radioligand was added to 1 mL of fresh human serum and incubated at 37 °C. At each given timepoint 25 µL of the mixture was loaded on to the column, which was then spun at 20,000 rcf for 1 min. The activity content on the column (free radioligand) and in the filtrate (protein bound fraction) was quantified in a γ-counter.

### Stability studies

Ligand stability in human blood serum (n = 2) and PBS (pH = 6.5) (n = 1) was determined for different time points (2 min, 30 min, 60 min, 120 min). Therefore, approx. 5 MBq of radioligand was added to 1 mL of fresh human serum at 37 °C or PBS at rt. At each given time point 100 µL of the mixture were transferred to a 1.5 mL Eppendorf tube containing 100 µL of acetonitrile. The vial was vortexed and centrifuged for 2 min at 14,000 1/min before 100 µL of the supernatant were diluted with 200 µL of water and injected into analytical radio-HPLC. The PBS solution was directly injected into the radio-HPLC. Ligand stability was determined by integrating the areas of the signals in the radio chromatogram in relation to the signal of the intact ligand.

Table 1 shows the results of the stability studies, the protein binding and the logarithmic partition coefficient. This data demonstrate that all compounds reveal a very hydrophilic character with high stability in serum and low serum protein binding up to 2 hours incubation time.

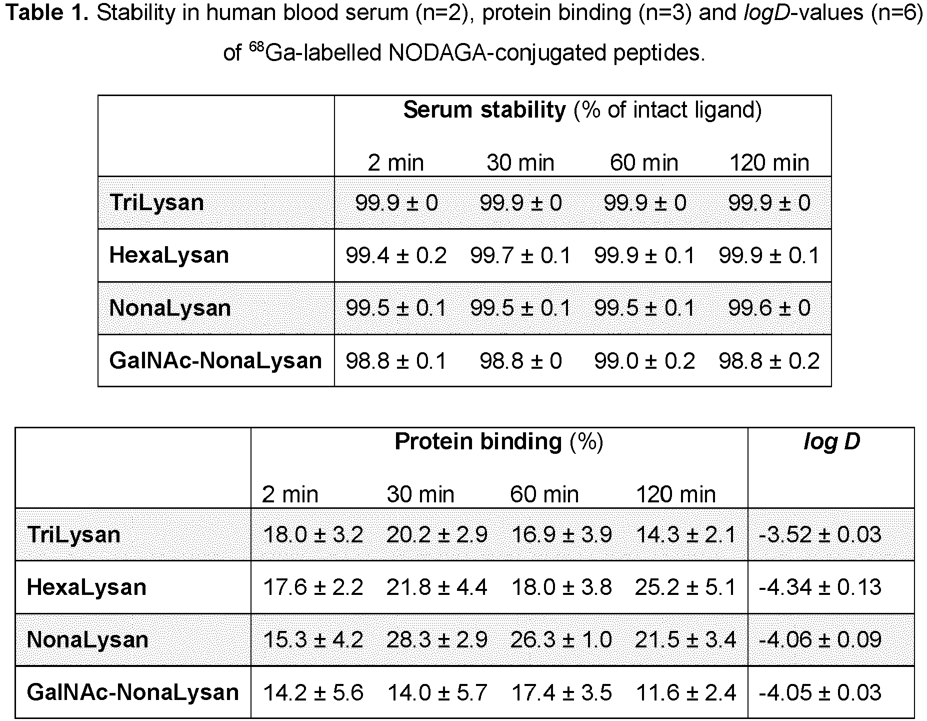

### Example 8: In vivo evaluation

### Biodistribution studies

All animal experiments were conducted in accordance with Austrian animal experiments law (BGBI. I Nr. 114/2012) and according to the institution's animal welfare standards as approved by the government of Austria (2022-0.311.708).

For biodistribution studies 6-week-old female BALB/c mice (n=3) were injected with 0.1 nmol (100 µL, approx. 1 MBq) of labelled compound via the lateral tail vein and sacrificed after 10, 30, or 60 min by cervical dislocation. The mice were dissected, blood and organs (spleen, pancreas, stomach, intestine, liver, kidney, heart, lung, muscle and bone) were weighed and their activity measured in a γ-counter. For blocking experiments, mice were co-injected with 27.7 µmοl *N*-acetylgalactose.

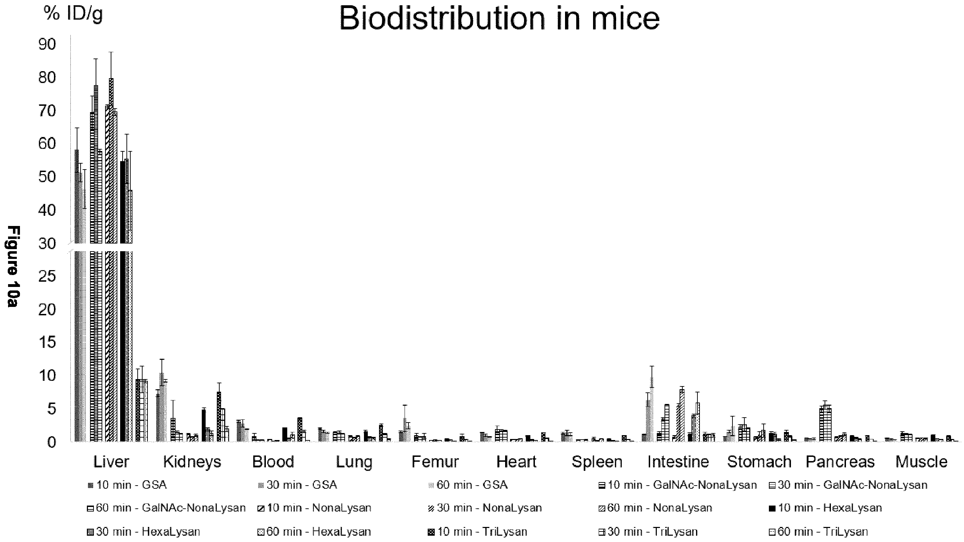

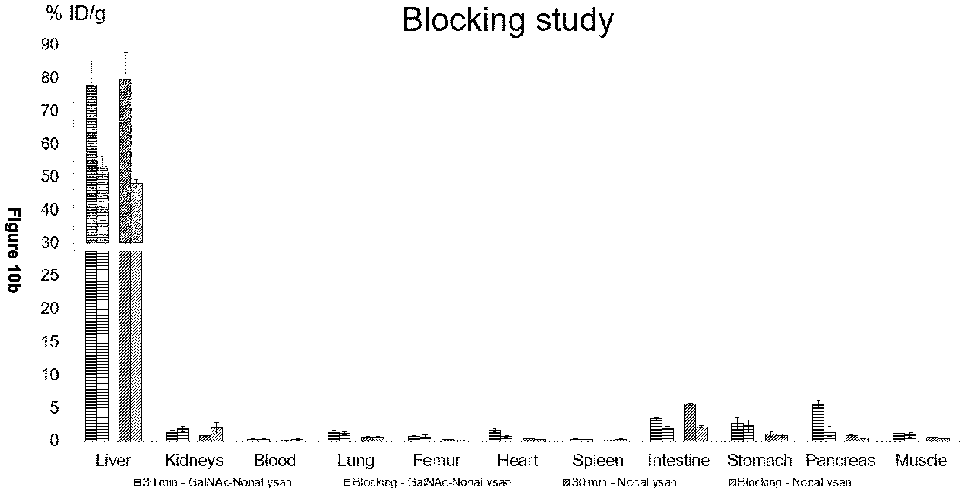

The results of the biodistribution studies are shown in Fig. 10a & b.

**Table 2: Biodistribution data of [⁶⁸Ga]Ga-TriLysan**

| Organ | 10 min | 30 min | 60 min |
|---|---|---|---|
| Liver | 9.45 ± 1.58 | 9.44 ± 1.99 | 9.19 ± 0.27 |
| Kidneys | 7.56 ± 1.35 | 4.99 ± 0.02 | 1.99 ± 0.34 |
| Blood | 3.64 ± 0.08 | 1.60 ± 0.17 | 0.27 ± 0.01 |
| Lung | 2.56 ± 0.17 | 1.15 ± 0.11 | 0.47 ± 0.06 |
| Femur | 0.89 ± 0.32 | 0.39 ± 0.02 | 0.11 ± 0.01 |
| Heart | 1.41 ± 0.01 | 0.63 ± 0.04 | 0.15 ± 0.01 |
| Spleen | 1.00 ± 0.01 | 0.42 ± 0.02 | 0.16 ± 0.00 |
| Intestine | 1.24 ± 0.26 | 1.13 ± 0.02 | 1.04 ± 0.26 |
| Stomach | 1.55 ± 0.24 | 0.88 ± 0.09 | 0.31 ± 0.05 |
| Pancreas | 0.97 ± 0.04 | 0.44 ± 0.00 | 0.17 ± 0.01 |
| Muscle | 0.90 ± 0.05 | 0.40 ± 0.04 | 0.12 ± 0.00 |

**Table 3: Biodistribution data of [⁶⁸Ga]Ga-HexaLysan**

| Organ | 10 min | 30 min | 60 min |
|---|---|---|---|
| Liver | 54.76 ± 3.02 | 55.46 ± 7.37 | 45.88 ± 11.77 |
| Kidneys | 4.83 ± 0.30 | 1.94 ± 0.22 | 1.34 ± 0.34 |
| Blood | 2.10 ± 0.04 | 0.63 ± 0.07 | 1.05 ± 0.41 |
| Lung | 1.61 ± 0.16 | 0.77 ± 0.11 | 0.62 ± 0.19 |
| Femur | 0.48 ± 0.06 | 0.33 ± 0.03 | 0.002 ± 0.001 |
| Heart | 0.94 ± 0.05 | 0.37 ± 0.09 | 0.25 ± 0.02 |
| Spleen | 0.50 ± 0.07 | 0.21 ± 0.06 | 0.17 ± 0.02 |
| Intestine | 1.19 ± 0.25 | 3.97 ± 0.28 | 5.94 ± 1.67 |
| Stomach | 1.29 ± 0.28 | 1.19 ± 0.28 | 0.40 ± 0.14 |
| Pancreas | 0.91 ± 0.05 | 0.69 ± 0.12 | 0.48 ± 0.13 |
| Muscle | 0.93 ± 0.12 | 0.57 ± 0.03 | 0.41 ± 0.04 |

**Table 4: Biodistribution data of [⁶⁸Ga]Ga-NonaLysan**

| Organ | 10 min | 30 min | 60 min |
|---|---|---|---|
| Liver | 71.23 ± 0.43 | 79.56 ± 7.96 | 69.64 ± 0.98 |
| Kidneys | 1.16 ± 0.12 | 0.83 ± 0.03 | 0.99 ± 0.15 |
| Blood | 0.43 ± 0.03 | 0.15 ± 0.04 | 0.22 ± 0.05 |
| Lung | 0.85 ± 0.10 | 0.70 ± 0.08 | 0.92 ± 0.10 |
| Femur | 0.26 ± 0.02 | 0.26 ± 0.08 | 0.23 ± 0.01 |
| Heart | 0.44 ± 0.04 | 0.44 ± 0.04 | 0.52 ± 0.07 |
| Spleen | 0.57 ± 0.16 | 0.17 ± 0.03 | 0.49 ± 0.03 |
| Intestine | 0.77 ± 0.17 | 5.61 ± 0.22 | 7.91 ± 0.49 |
| Stomach | 0.73 ± 0.16 | 1.15 ± 0.44 | 1.78 ± 0.98 |
| Pancreas | 0.75 ± 0.07 | 0.91 ± 0.05 | 1.16 ± 0.19 |
| Muscle | 0.61 ± 0.07 | 0.66 ± 0.03 | 0.59 ± 0.09 |

Fig. 10 and tables 2- 4 show an increasing activity accumulation in the kidneys in the series [⁶⁸Ga]Ga-TriLysan, [⁶⁸Ga]Ga-HexaLysan and [⁶⁸Ga]Ga-NonaLysan. The low accumulation of [⁶⁸Ga]Ga-TriLysan can be explained by the unfavorable presentation of the 3 galactose units in this compound. Increasing the potential binding motifs to 6 or 9 galactose units results also in an increase in the tracer uptake. [⁶⁸Ga]Ga-NonaLysan even outperforms the accumulation in the liver found for the gold standard ^{99m}Tc-GSA. But not only the receptor-specific uptake in the liver is higher, also the elimination in all non-target organs is faster/higher leading to very high liver-to-organ ratios. The only organ with increasing activity concentration over time is the intestine, over which some of the tracer is excreted. But even there, activity concentration is lower as found for ^{99m}Tc-GSA. We further tested compounds wherein the sugar residue Gal was replaced with the N-acetylated form GaINAc. We found that compounds having GalNAc as the sugar residue perform equally well as the corresponding derivatives having Gal as the sugar residue.

### PET/MR imaging

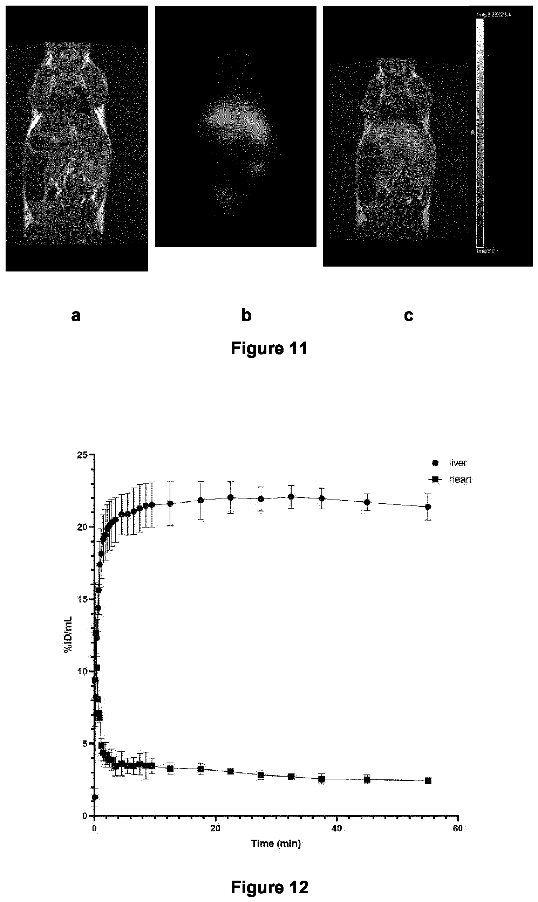

All animal experiments were performed according to the German animal welfare act and approved by the local authorities. All male C57BU6J mice (n=3 per group) were purchased from Janvier Lab at 6 weeks. Mice were kept under 1.5% isoflurane in oxygen during the measurements. Approximately 1 MBq of the radiotracer was injected intravenously per mouse and 1 hour dynamic PET was performed with an Inveon microPET system (Inveon D-PET, Siemens, Knoxville, TN, USA). Then the mice were transferred to a 7 Tesla BioSpec 70/30 USR (Bruker Biospin MRI GmbH) and a 15 min MR anatomical scan using T2-weighted 3-dimensional turbo spin-echo sequence was acquired. An Inveon Acquisition Workplace was used to reconstruct the acquired images using ordered subset expectation maximization 3D (OSEM 3D) and framing the dynamics. The reconstructed image analysis was performed by using an Inveon Research Workplace (IRW). After co-registering acquired MR images with PET images, regions of interest (ROIs) were drawn on liver and heart and time activity curves (TACs) were quantified (Fig.12).

Representative frames of dynamic PET/MR imaging over 60 min after injection of [⁶⁸Ga]Ga-NODAGA-NonaLysan demonstrated an intensive liver signal and low background activity in the rest of the body (Fig. 11).

## Claims

1. A compound of formula (I), or a pharmaceutically acceptable salt thereof, 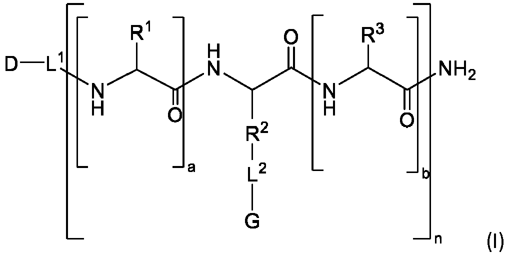 wherein
D is a chelating group or a prosthetic group or a [¹⁸F]F accepting group;
each instance of R¹ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or R¹ forms a 5-membered heterocycle with the alpha-nitrogen, the heterocycle containing up to one additional heteroatom selected from O, N and S;
each instance of R² is independently selected from the group consisting of ^{#}-NH-(C(R⁴)₂)_{d}-, and ^{#}-(C=O)-(C(R⁴)₂)_{d}-,
wherein ^{#} designates the attachment point to L²;
each instance of R³ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}-, NH₂-(C(R⁴)₂)_{d}-, NH₂-C(N=H)-NH-(C(R⁴)₂)_{d}-, NH₂-(C=O)-(C(R⁴)₂)_{d}-, (COOH)-(C(R⁴)₂)_{d}- and R⁵-(C(R⁴)₂)_{d}-, or Reforms a 5-membered heterocycle with the alpha-nitrogen, the heterocycle containing up to one additional heteroatom selected from O, N and S;
each instance of R⁴ is independently selected from the group consisting of H, C₁₋₄-alkyl, and OH;
each instance of R⁵ is independently selected from the group consisting of 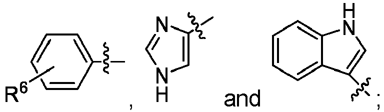
R⁶ is selected from the group consisting of H, Hal and OH;
L¹ is a linker selected from the group consisting of *-N(H)-(CH₂)ₓ-C(O)-, aspartic acid, glutamic acid, lysine, *-N(H)-(CH₂-CH₂-O)_{y}-(CH₂)_{z}-C(O)-, 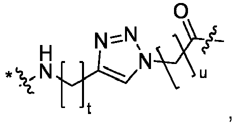 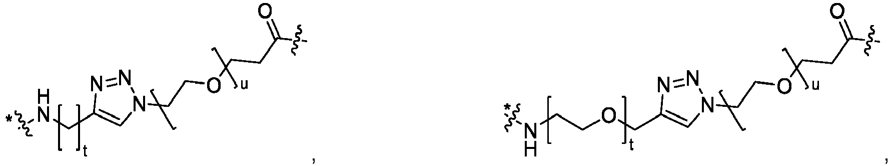 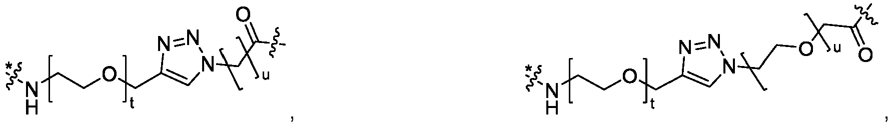 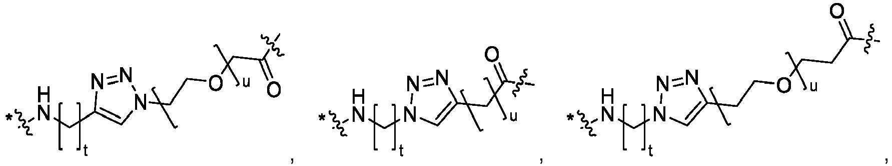 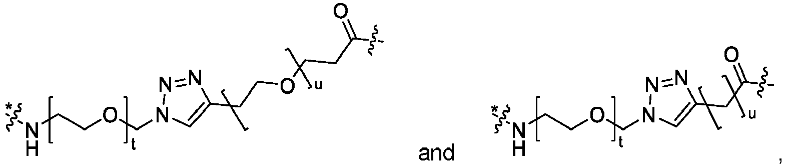 wherein
* designates the attachment point to D;
L² is a linker selected from the group consisting of 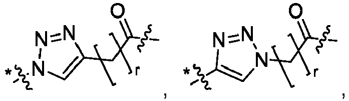 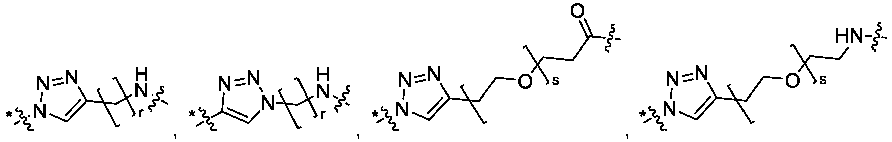 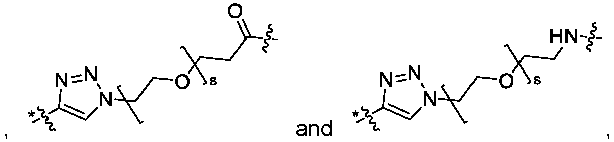 wherein * designates the attachment point to G;
G is Gal, GaINAc, or Lac;
a is 0-3;
b is 1-6; with the proviso that a + b ≤ 6;
d is independently selected from 0-6;
n is 3-15;
r is independently selected from 1-6;
s is 1-6;
u is independently selected from 1-6;
t is independently selected from 3-6;
x is 1-6;
y is 1-6; and
z is 1-2.

2. The compound according to claim 1, or a pharmaceutically salt thereof, wherein D is a chelating group selected from the group consisting of NODAGA, NOTA, DOTAGA, DO3A, HBED, DTPA, DFO, FusC, DafC, CNAAZTA, NOPO, TRAP, DOTPI, DOTAZA, sacrophagine-based chelators, and TE2A derivatives such as CB-TE2A.

3. The compound according to claim 1 or 2, wherein D is a chelating group selected from the group consisting of 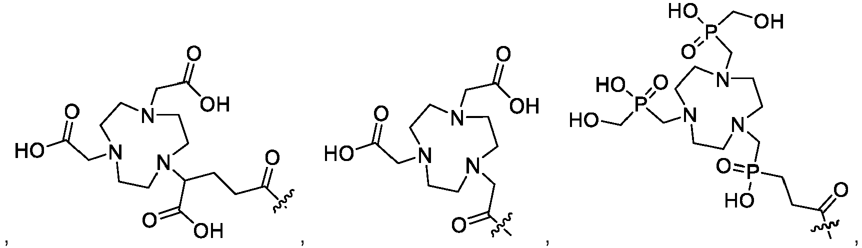 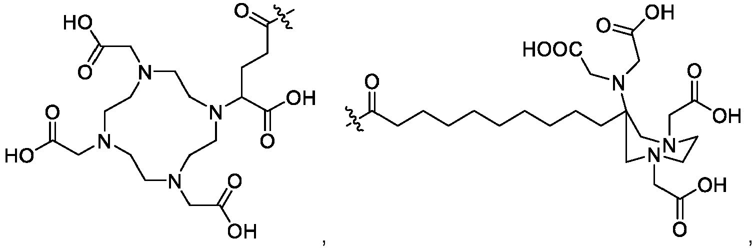 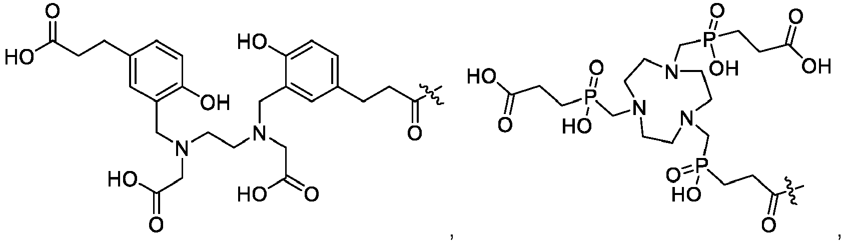 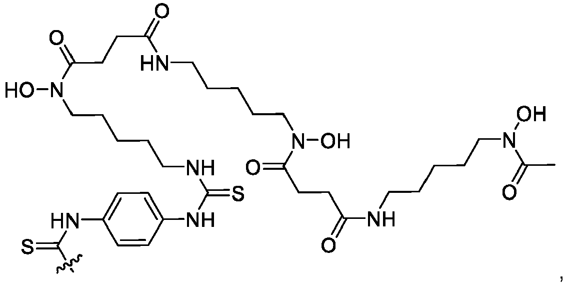 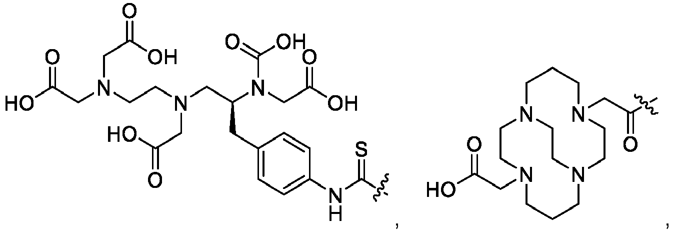 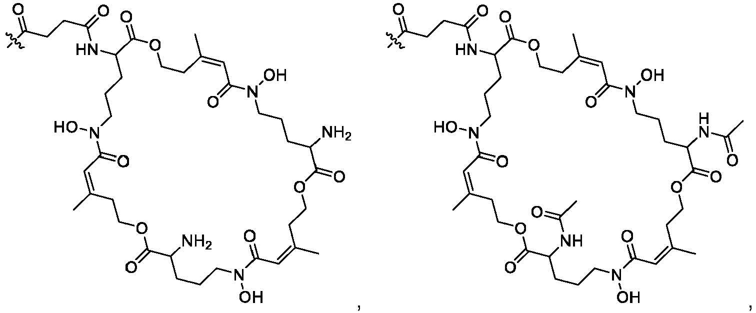 and

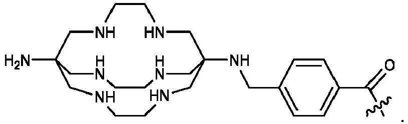

4. The compound according to any one of claims 1-4, wherein each instance of R¹ or R³ is independently selected from the group consisting of H, CH₃-(C(R⁴)₂)_{d}-, HS-(C(R⁴)₂)_{d}-, CH₃-S-(C(R⁴)₂)_{d}-, HO-(C(R⁴)₂)_{d}- and NH₂-(C(R⁴)₂)_{d}-; wherein R⁴ is H, CH₃ or OH and d is 0-2.

5. The compound according to any one of claims 1-5, wherein n is 3, 6, 9, 12 or 15.

6. The compound according to any one of claims 1-5, wherein
D is a chelating group or a prosthetic group or a [¹⁸F]F accepting group;
R¹ is independently from each other selected from the group consisting of H and CH₃-;
R² is independently from each other selected from the group consisting of ^{#}-NH-(C(R⁴)₂)_{d}-, wherein R⁴ is H, d is 3-4 and ^{#} designates the attachment point to L²;
R³ is independently from each other selected from the group consisting of H and CH₃-;
L¹ is *-N(H)-(CH₂)ₓ-C(O)- or *-N(H)-(CH₂-CH₂-O)_{y}-(CH₂)_{z}-C(O)-, wherein x is 2-4, y is 1-4 and z is 1-2 and * designates the attachment point to D; L² is 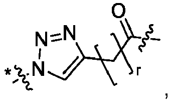 wherein r is 3-5 and * designates the attachment point to G;
G is Gal, GaINAc, or Lac; and
n is 3, 6, or 9.

7. The compound according to any one of claims 1-4, or a pharmaceutically acceptable salt thereof, wherein the compound is selected from 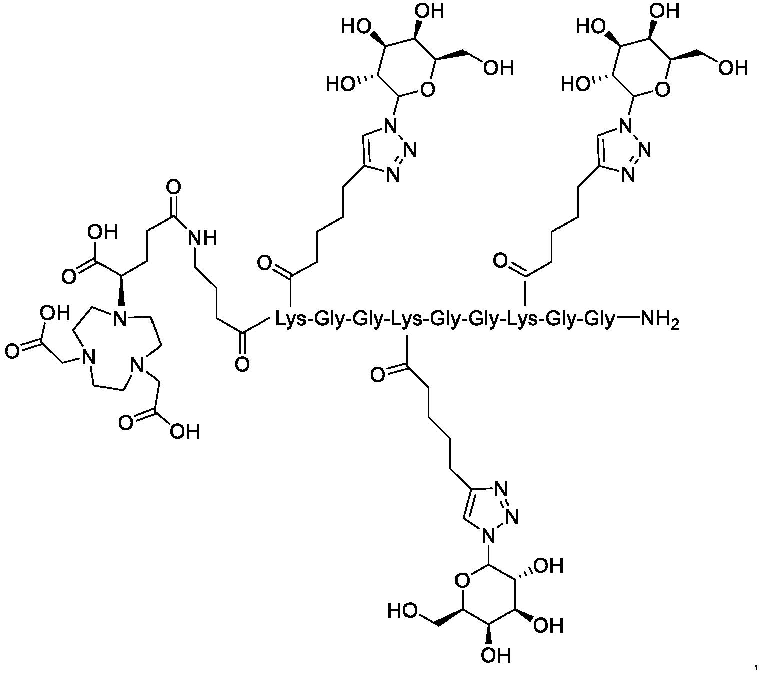 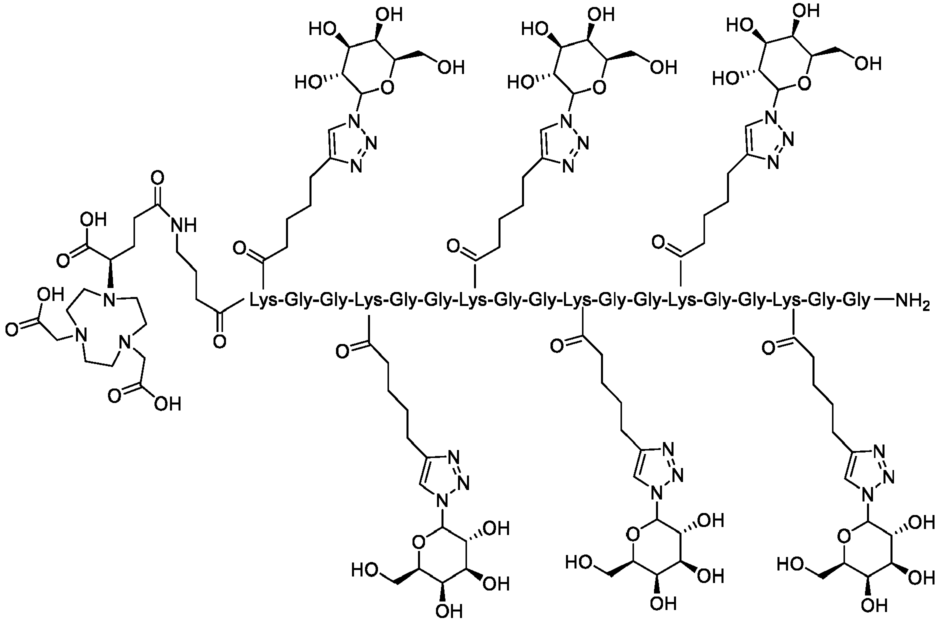 and

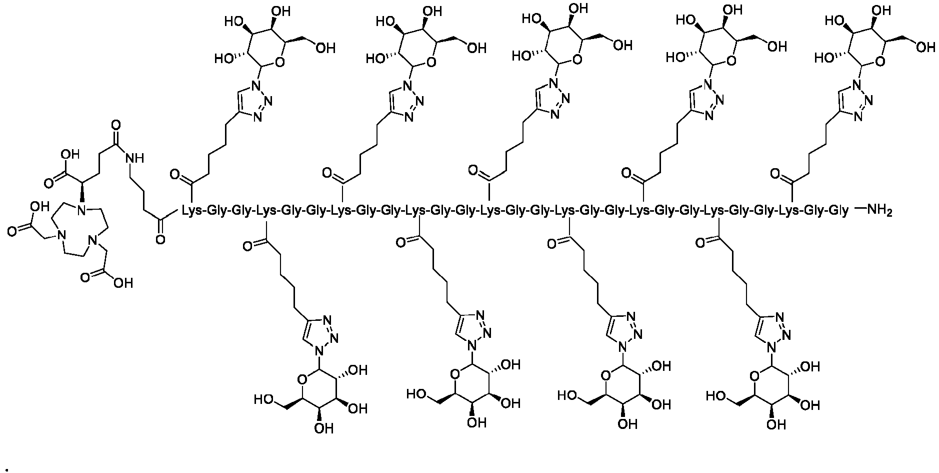

8. The compound according to any one of claims 1-4, or a pharmaceutically acceptable salt thereof, wherein D is
a) a chelating group which chelates [⁶⁸Ga]Ga, [¹⁸F]AlF, [⁸⁹Zr]Zr, [⁶⁴Cu]Cu or [⁸⁶Y]Y; or
b) a prosthetic group which is labelled with radionuclides of halogens selected from [¹²⁴I]I, [¹²³I]I, [⁷⁵Br]Br, [⁷⁶Br]Br or [¹⁸F]F; or
c) a [¹⁸F]F accepting group which is modified with [¹⁸F]F.

9. A method of producing the compound according to any one of claims 1-8, comprising synthesizing the compound.

10. A pharmaceutical or diagnostic composition comprising the compound according to any one of claims 1-6 and at least one additive.

11. A method of imaging hepatocytes, comprising a) contacting the hepatocyte with a compound according to any one of claims 1-8, and b) visualizing the compound that is in contact with the hepatocyte.

12. A method of determining functional hepatic reserve, comprising a) contacting the liver with the compound according to any one of claims 1-8, and b) visualizing the compound that is in contact with the liver.

13. The method according to claim 11 or 12, wherein the compound is visualized by positron emission tomography (PET).

14. Use of the compound according to any one of claims 1-8 for imaging hepatocytes.

15. Use of the compound according to any one of claims 1-8 for determining functional hepatic reserve.
